# EUROPEAN PATENT APPLICATION

(11) **EP 3 459 367 A1**
(43) Date of publication of application: **27.03.2019**
(21) Application number: 17810350.3
(22) Date of filing: 07.06.2017
(51) Int. Cl.: A23L 33/10, A23L 33/115, A23L 33/15, A23L 33/155, A61K 31/122, A61K 31/355, A61K 36/05, A61K 36/63, A61P 25/00, A61P 25/18, A61P 25/20, A61P 25/24, A61P 25/28, A61P 43/00

(54) **FOOD OR BEVERAGE COMPOSITION CONTAINING ASTAXANTHIN**

(30) Priority: 08.06.2016 JP 2016114469; 22.12.2016 JP 2016248990
(71) Applicant: AstaReal Co., Ltd., Nakaniikawa-gun, Toyama 930-0397 (JP)
(72) Inventor: TOMINAGA Kumi, Nakaniikawa-gun Toyama 930-0397 (JP); HONGO Nobuko, Nakaniikawa-gun Toyama 930-0397 (JP); FUJISHITA Mayuko, Nakaniikawa-gun Toyama 930-0397 (JP)
(74) Representative: Prüfer & Partner mbB Patentanwälte · Rechtsanwälte
(86) International application number: PCT/JP2017/021153
(87) International publication number: WO 2017/213176

(57) **Abstract**

Provided are a food-and-drink composition, food-and-drink, and so on containing astaxanthin and/or astaxanthin-containing extract as an active ingredient. The food-and-drink composition, food-and-drink, and so on containing astaxanthin and/or astaxanthin-containing extract as an active ingredient exert actions and effects to improve dull-headedness, improve decreased concentration, improve decreased motivation, improve depressed mood, resolve frustration, reduce feeling of body heaviness, and improve decreased vigor and/or activity, in a healthy human male or female having a sense of fatigue, in particular, a sense of fatigue caused by a mental load or a sense of fatigue caused by a mental load and a physical load.

## Description

### Technical Field

The present invention relates to a food-and-drink composition containing astaxanthin and/or astaxanthin-containing extract as an active ingredient, more specifically, to a food-and-drink composition containing astaxanthin and/or astaxanthin-containing extract as an active ingredient as one or more selected from food-and-drink compositions for improving dull-headedness, for improving decreased concentration, for improving decreased motivation, for improving depressed mood, for resolving frustration, for reducing feeling of body heaviness, for improving decreased vigor and/or activity, for enhancing feeling of friendliness, for improving the quality of sleep, for resolving physical stress, for preventing accumulation of mental stress, for preventing accumulation of fatigue, and for preventing immunosuppression, in a human having a sense of fatigue, in particular, a sense of fatigue caused by a mental load or a sense of fatigue caused by a mental load and a physical load.

### Background Art

Astaxanthin (astaxanthine, 3,3'-dihydroxy-β,β-carotene-4,4'-dione) is one of carotenoids including β-carotene from carrots and lycopene from tomatoes, and is a reddish orange pigment substance which is classified as a xanthophyll and has long been used for foods. Astaxanthin is widely present in nature, and can be found, as a familiar example, in a shell of a crustacean as well as the body surface of a red seabream and red portions in the muscle of a salmonid and the like, which feed crustaceans. Astaxanthin exists in three forms: a free form, a monoester form, and a diester form.

Naturally-derived astaxanthin and synthetic astaxanthin are used in the industrial and commercial fields, and examples of the naturally-derived astaxanthin include natural astaxanthin derived from Haematococcus algae, which is supplied to the food market through the world's first industrial indoor tank culture of Haematococcus algae by AstaReal Holdings Co., Ltd. belonging to Fuji Chemical Group at present.

Astaxanthin has long been used for a pigment as a food additive and a pigmentation agent for cultured fish. Since astaxanthin was found to have excellent antioxidative effect 1000 times as high as that of vitamin E, astaxanthin has been used for pharmaceutical products, health foods including supplements, basic skin care products, and so on. Thereafter, various actions and effects were found for astaxanthin, and astaxanthin is finding wider applications.

Examples of such actions and effects include antiinflammatory action, anti-arteriosclerosis action, improvement of blood flow, improvement of fatigue, improvement of fatigue accompanied by decreased motivation, and improvement of a state with appreciable fatigue (Patent Literature 1); improvement of brain dysfunction and effect similar to that by exercise to relieve stress and provide better feeling (Patent Literature 2); suppression of generation of active oxygen in the brain (Patent Literature 3); enhancement of cognitive-behavioral performance (Patent Literature 4); anti-anxiety action and anti-fatigue effect (Patent Literature 5); deodorization of excrement, improvement of sleep, improvement of sensitivity, and improvement of vision Patent Literature 6); and prevention or improvement of fatigue symptoms due to stress (Patent Literature 7). Particularly in the field of foods, the recent emergence of foods for specified health use (FOSHU), foods with nutrient function claims, and foods with function claims has generated demand for development of specific applications meeting needs of particular consumers for pharmaceutical products.

### Citation List

### Patent Literature

Patent Literature 1: Japanese Patent Laid-Open No. 2006-347927
Patent Literature 2: Japanese Patent Laid-Open No. 2007-126455
Patent Literature 3: Japanese Patent Laid-Open No. 2007-314436
Patent Literature 4: Japanese Patent Laid-Open No. 2010-270095
Patent Literature 5: Japanese Patent Laid-Open No. 2012-025712
Patent Literature 6: International Publication No. WO 2005/058064
Patent Literature 7: Japanese Patent Laid-Open No. 1997-124470

### Summary of Invention

### Technical Problem

In Patent Literature 1, as an anti-fatigue study, a human was subjected to treadmill exercise followed by measurement of the blood lactic acid level to confirm that the human was in a state of fatigue, and with the confirmation it was demonstrated that administration of astaxanthin can ameliorate the state of fatigue and decrease the sense of fatigue. However, Patent Literature 1 does not demonstrate that ingestion of astaxanthin by a human still having a sense of fatigue can improve dull-headedness, improve decreased concentration, improve decreased motivation, improve depressed mood, resolve frustration, reduce feeling of body heaviness, improve decreased vigor and/or activity, enhance feeling of friendliness, improve or enhance the quality of sleep with respect to, for example, sleepiness on rising, initiation and maintenance of sleep, frequent dreaming, and sleep length, reduce, improve, or resolve physical stress, prevent accumulation of mental stress, prevent accumulation of fatigue, and prevent immunosuppression. Patent Literature 1 recites "fatigue is a disease associated with a sense of fatigue" (paragraph [0002]), and the presence or absence of a state of "fatigue" is determined, for example, through measurement of the blood lactic acid level, as disclosed in Test Example 3 in Patent Literature 1. However, the presence or absence of a "sense of fatigue" is determined, for example, through VAS (Visual Analog Scale), as described herein in Example. Hence, in the case that a certain mental or physical load is applied to subjects as in Example in the present specification, the results are different among the subjects, and some subjects "are not in a state of fatigue but have a sense of fatigue" and other subjects "are in a state of fatigue but do not have a sense of fatigue". Thus, it is not necessarily the case that "fatigue is associated with a sense of fatigue".

Patent Literature 2 demonstrates that administration of astaxanthin to a mouse in a depressed state can improve the depressed state, or administration of astaxanthin can improve or prevent damage caused by aging to the brain of a mouse, and does not demonstrate, as with the case of Patent Literature 1, that ingestion of astaxanthin can improve dull-headedness, improve decreased concentration, improve decreased motivation, improve depressed mood, resolve frustration, reduce feeling of body heaviness, improve decreased vigor and/or activity, enhance feeling of friendliness, improve or enhance the quality of sleep with respect to, for example, sleepiness on rising, initiation and maintenance of sleep, frequent dreaming, and sleep length, reduce, improve, or resolve physical stress, prevent accumulation of mental stress, prevent accumulation of fatigue, and prevent immunosuppression, in a human having a sense of fatigue, in particular, a sense of fatigue caused by a mental load or a sense of fatigue caused by a mental load and a physical load.

Patent Literature 3 demonstrates that administration of astaxanthin can effectively prevent oxidative damage caused to the brain of a rat, can effectively prevent and treat cerebral infarction, can prevent various diseases due to active oxygen in the brain, and can inhibit aging of the brain, and does not demonstrate, as with the case of Patent Literatures 1 and 2, that ingestion of astaxanthin can improve dull-headedness, improve decreased concentration, improve decreased motivation, improve depressed mood, resolve frustration, reduce feeling of body heaviness, improve decreased vigor and/or activity, enhance feeling of friendliness, improve or enhance the quality of sleep with respect to, for example, sleepiness on rising, initiation and maintenance of sleep, frequent dreaming, and sleep length, reduce, improve, or resolve physical stress, prevent accumulation of mental stress, prevent accumulation of fatigue, and prevent immunosuppression, in a human having a sense of fatigue, in particular, a sense of fatigue caused by a mental load or a sense of fatigue caused by a mental load and a physical load.

Patent Literature 4 demonstrates that ingestion of astaxanthin can enhance cognitive-behavioral performance, in which higher brain functions are involved, in an elderly individual, and as a result can enhance the physical-exercise ability of the elderly individual, and does not demonstrate, as with the case of Patent Literatures 1 to 3, that ingestion of astaxanthin can improve dull-headedness, improve decreased concentration, improve decreased motivation, improve depressed mood, resolve frustration, reduce feeling of body heaviness, improve decreased vigor and/or activity, enhance feeling of friendliness, improve or enhance the quality of sleep with respect to, for example, sleepiness on rising, initiation and maintenance of sleep, frequent dreaming, and sleep length, reduce, improve, or resolve physical stress, prevent accumulation of mental stress, prevent accumulation of fatigue, and prevent immunosuppression, in a human having a sense of fatigue, in particular, a sense of fatigue caused by a mental load or a sense of fatigue caused by a mental load and a physical load.

Patent Literature 5 demonstrates that administration of astaxanthin for mice provides anti-anxiety effects, i.e., effects to treat, alleviate, or prevent the condition of anxiety such as neuropathy, mood disorders, personality disorders, behavior disorders, and sleep disorders, and does not demonstrate, as with the case of Patent Literatures 1 to 4, that ingestion of astaxanthin can improve dull-headedness, improve decreased concentration, improve decreased motivation, improve depressed mood, resolve frustration, reduce feeling of body heaviness, improve decreased vigor and/or activity, enhance feeling of friendliness, improve or enhance the quality of sleep with respect to, for example, sleepiness on rising, initiation and maintenance of sleep, frequent dreaming, and sleep length, reduce, improve, or resolve physical stress, prevent accumulation of mental stress, prevent accumulation of fatigue, and prevent immunosuppression, in a human having a sense of fatigue, in particular, a sense of fatigue caused by a mental load or a sense of fatigue caused by a mental load and a physical load.

Patent Literature 6 demonstrates that administration of astaxanthin provides excretion-deodorizing effect, sleep-improving effect, sensitivity-improving effect, and vision-improving effect for dogs, and does not demonstrate, as with the case of Patent Literatures 1 to 5, that ingestion of astaxanthin by a human having a sense of fatigue can improve dull-headedness, improve decreased concentration, improve decreased motivation, improve depressed mood, resolve frustration, reduce feeling of body heaviness, improve decreased vigor and/or activity, enhance feeling of friendliness, improve or enhance the quality of sleep with respect to, for example, sleepiness on rising, initiation and maintenance of sleep, frequent dreaming, and sleep length, reduce, improve, or resolve physical stress, prevent accumulation of mental stress, prevent accumulation of fatigue, and prevent immunosuppression, in a human having a sense of fatigue, in particular, a sense of fatigue caused by a mental load or a sense of fatigue caused by a mental load and a physical load.

Patent Literature 7 shows the test result that administration of astaxanthin provided suppression effect against restraint stress for mice to conclude that administration of astaxanthin inhibits, prevents, or improves the symptoms themselves of physical fatigue caused by physical stress and mental fatigue caused by mental stress, or health disorders due to them, and does not demonstrate, as with the case of Patent Literatures 1 to 6, that ingestion of astaxanthin can improve dull-headedness, improve decreased concentration, improve decreased motivation, improve depressed mood, resolve frustration, reduce feeling of body heaviness, improve decreased vigor and/or activity, enhance feeling of friendliness, improve or enhance the quality of sleep with respect to, for example, sleepiness on rising, initiation and maintenance of sleep, frequent dreaming, and sleep length, reduce, improve, or resolve physical stress, prevent accumulation of mental stress, prevent accumulation of fatigue, and prevent immunosuppression, in a human having a sense of fatigue, in particular, a sense of fatigue caused by a mental load or a sense of fatigue caused by a mental load and a physical load.

In view of the circumstances, an object of the present invention is to provide a food-and-drink composition, food-and-drink, and so on containing astaxanthin and/or astaxanthin-containing extract as an active ingredient for a human having a sense of fatigue, in particular, a sense of fatigue caused by a mental load or a sense of fatigue caused by a mental load and a physical load.

### Solution to Problem

The present inventors diligently studied and found that astaxanthin and/or astaxanthin-containing extract ingested by a healthy human male or female having a sense of fatigue, in particular, a sense of fatigue caused by a mental load or a sense of fatigue caused by a mental load and a physical load exerts actions and effects to improve dull-headedness, improve decreased concentration, improve decreased motivation, improve depressed mood, resolve frustration, reduce feeling of body heaviness, improve decreased vigor and/or activity, enhance feeling of friendliness, improve or enhance the quality of sleep with respect to, for example, sleepiness on rising, initiation and maintenance of sleep, frequent dreaming, and sleep length, reduce, improve, or resolve physical stress, prevent accumulation of mental stress, prevent accumulation of fatigue, and prevent immunosuppression, and further found that these actions and effects are exerted without any antioxidative effect in a human, and thus completed each of the following inventions.
(1) A food-and-drink composition containing astaxanthin and/or astaxanthin-containing extract as an active ingredient as one or more selected from the following (a), (b), (c), (d), (e), (f), and (g):
   (a) a food-and-drink composition for improving dull-headedness in a human having a sense of fatigue;
   (b) a food-and-drink composition for improving decreased concentration in a human having a sense of fatigue;
   (c) a food-and-drink composition for improving decreased motivation in a human having a sense of fatigue;
   (d) a food-and-drink composition for improving depressed mood in a human having a sense of fatigue;
   (e) a food-and-drink composition for resolving frustration in a human having a sense of fatigue;
   (f) a food-and-drink composition for reducing feeling of body heaviness in a human having a sense of fatigue; and
   (g) a food-and-drink composition for improving decreased vigor and/or activity in a human having a sense of fatigue.
(2) A food-and-drink composition containing astaxanthin and/or astaxanthin-containing extract as an active ingredient as one or more selected from the following (a), (b), (c), (d), and (e):
   (a) a food-and-drink composition for improving dull-headedness in a human having a sense of fatigue to be ingested by (administered to) a subject in need thereof in a dosage achieving a human blood astaxanthin level equal to approximately 0 ng/mL to approximately 160 ng/mL in terms of a free form of astaxanthin or an equivalent dosage thereof;
   (b) a food-and-drink composition for improving decreased concentration in a human having a sense of fatigue to be ingested by (administered to) a subject in need thereof in a dosage achieving a human blood astaxanthin level equal to approximately 0 ng/mL to approximately 160 ng/mL in terms of a free form of astaxanthin or an equivalent dosage thereof;
   (c) a food-and-drink composition for improving decreased motivation in a human having a sense of fatigue to be ingested by (administered to) a subject in need thereof in a dosage achieving a human blood astaxanthin level equal to approximately 0 ng/mL to approximately 160 ng/mL in terms of a free form of astaxanthin or an equivalent dosage thereof;
   (d) a food-and-drink composition for improving depressed mood in a human having a sense of fatigue to be ingested by (administered to) a subject in need thereof in a dosage achieving a human blood astaxanthin level equal to approximately 0 ng/mL to approximately 160 ng/mL in terms of a free form of astaxanthin or an equivalent dosage thereof; and
   (e) a food-and-drink composition for resolving frustration in a human having a sense of fatigue to be ingested by (administered to) a subject in need thereof in a dosage achieving a human blood astaxanthin level equal to approximately 0 ng/mL to approximately 160 ng/mL in terms of a free form of astaxanthin or an equivalent dosage thereof.
(3) A food-and-drink composition containing astaxanthin and/or astaxanthin-containing extract as an active ingredient as one or more selected from the following (a), (b), (c), (d), and (e):
   (a) a food-and-drink composition for improving dull-headedness in a human having a sense of fatigue to be ingested by (administered to) a subject in need thereof in a dosage equal to approximately 0.1 mg to approximately 0.3 mg per kg of body weight per day in terms of a free form of astaxanthin or an equivalent dosage thereof;
   (b) a food-and-drink composition for improving decreased concentration in a human having a sense of fatigue to be ingested by (administered to) a subject in need thereof in a dosage equal to approximately 0.1 mg to approximately 0.3 mg per kg of body weight per day in terms of a free form of astaxanthin or an equivalent dosage thereof;
   (c) a food-and-drink composition for improving decreased motivation in a human having a sense of fatigue to be ingested by (administered to) a subject in need thereof in a dosage equal to approximately 0.1 mg to approximately 0.3 mg per kg of body weight per day in terms of a free form of astaxanthin or an equivalent dosage thereof;
   (d) a food-and-drink composition for improving depressed mood in a human having a sense of fatigue to be ingested by (administered to) a subject in need thereof in a dosage equal to approximately 0.1 mg to approximately 0.3 mg per kg of body weight per day in terms of a free form of astaxanthin or an equivalent dosage thereof; and
   (e) a food-and-drink composition for resolving frustration in a human having a sense of fatigue to be ingested by (administered to) a subject in need thereof in a dosage equal to approximately 0.1 mg to approximately 0.3 mg per kg of body weight per day in terms of a free form of astaxanthin or an equivalent dosage thereof.
(4) The food-and-drink composition according to any one of (1) to (3), wherein the sense of fatigue is a sense of fatigue caused by a mental load or a sense of fatigue caused by a mental load and a physical load.
(5) The food-and-drink composition according to any one of (1) to (4), further containing at least any of tocotrienol and olive oil.
(6) The food-and-drink composition according to any one of (1) to (5), wherein the astaxanthin and/or astaxanthin-containing extract is astaxanthin and/or astaxanthin-containing extract obtained by crashing Haematococcus algae.
(7) The food-and-drink composition according to any one of (1) to (6), wherein the human is a healthy human.
(8) A food-and-drink containing astaxanthin and/or astaxanthin-containing extract as an active ingredient as one or more selected from the following (a), (b), (c), (d), (e), (f), and (g):
   (a) a food-and-drink for improving dull-headedness in a human having a sense of fatigue;
   (b) a food-and-drink for improving decreased concentration in a human having a sense of fatigue;
   (c) a food-and-drink for improving decreased motivation in a human having a sense of fatigue;
   (d) a food-and-drink for improving depressed mood in a human having a sense of fatigue;
   (e) a food-and-drink for resolving frustration in a human having a sense of fatigue;
   (f) a food-and-drink for reducing feeling of body heaviness in a human having a sense of fatigue; and
   (g) a food-and-drink for improving decreased vigor and/or activity in a human having a sense of fatigue.
(9) A pharmaceutical composition containing astaxanthin and/or astaxanthin-containing extract as an active ingredient as one or more selected from the following (a), (b), (c), (d), (e), (f), and (g):
   (a) a pharmaceutical composition for improving dull-headedness in a human having a sense of fatigue;
   (b) a pharmaceutical composition for improving decreased concentration in a human having a sense of fatigue;
   (c) a pharmaceutical composition for improving decreased motivation in a human having a sense of fatigue;
   (d) a pharmaceutical composition for improving depressed mood in a human having a sense of fatigue;
   (e) a pharmaceutical composition for resolving frustration in a human having a sense of fatigue;
   (f) a pharmaceutical composition for reducing feeling of body heaviness in a human having a sense of fatigue; and
   (g) a pharmaceutical composition for improving decreased vigor and/or activity in a human having a sense of fatigue.
(10) An agent containing astaxanthin and/or astaxanthin-containing extract as an active ingredient as one or more selected from the following (a), (b), (c), (d), (e), (f), and (g):
   (a) an agent for improving dull-headedness in a human having a sense of fatigue;
   (b) an agent for improving decreased concentration in a human having a sense of fatigue;
   (c) an agent for improving decreased motivation in a human having a sense of fatigue;
   (d) an agent for improving depressed mood in a human having a sense of fatigue;
   (e) an agent for resolving frustration in a human having a sense of fatigue;
   (f) an agent for reducing feeling of body heaviness in a human having a sense of fatigue; and
   (g) an agent for improving decreased vigor and/or activity in a human having a sense of fatigue.
(11) Astaxanthin and/or an astaxanthin-containing extract for use for at least any application of the following (a), (b), (c), (d), (e), (f), and (g):
   (a) improvement of dull-headedness in a human having a sense of fatigue;
   (b) improvement of decreased concentration in a human having a sense of fatigue;
   (c) improvement of decreased motivation in a human having a sense of fatigue;
   (d) improvement of depressed mood in a human having a sense of fatigue;
   (e) resolution of frustration in a human having a sense of fatigue;
   (f) reduction of feeling of body heaviness in a human having a sense of fatigue; and
   (g) improvement of decreased vigor and/or activity in a human having a sense of fatigue.
(12) The astaxanthin and/or astaxanthin-containing extract according to (11), for use as a food-and-drink.
(13) Astaxanthin and/or an astaxanthin-containing extract for use as a pharmaceutical product for at least any application of the following (a), (b), (c), (d), (e), (f), and (g):
   (a) improvement of dull-headedness in a human having a sense of fatigue;
   (b) improvement of decreased concentration in a human having a sense of fatigue;
   (c) improvement of decreased motivation in a human having a sense of fatigue;
   (d) improvement of depressed mood in a human having a sense of fatigue;
   (e) resolution of frustration in a human having a sense of fatigue;
   (f) reduction of feeling of body heaviness in a human having a sense of fatigue; and
   (g) improvement of decreased vigor and/or activity in a human having a sense of fatigue.
(14) A method for improving, reducing or resolving at least any of the following (a), (b), (c), (d), (e), (f), and (g) through administration (ingestion) of astaxanthin:
   (a) dull-headedness in a human having a sense of fatigue;
   (b) decreased concentration in a human having a sense of fatigue;
   (c) decreased motivation in a human having a sense of fatigue;
   (d) depressed mood in a human having a sense of fatigue;
   (e) frustration in a human having a sense of fatigue;
   (f) feeling of body heaviness in a human having a sense of fatigue; and
   (g) decreased vigor and/or activity in a human having a sense of fatigue.

### Advantageous Effects of Invention

The food-and-drink composition containing astaxanthin and/or astaxanthin-containing extract as an active ingredient according to the present invention can provide a food-and-drink composition, food-and-drink, pharmaceutical composition, and agent to exert actions and effects to improve dull-headedness, improve decreased concentration, improve decreased motivation, improve depressed mood, resolve frustration, reduce feeling of body heaviness, improve decreased vigor and/or activity, enhance feeling of friendliness, improve or enhance the quality of sleep with respect to, for example, sleepiness on rising, initiation and maintenance of sleep, frequent dreaming, and sleep length, reduce, improve, or resolve physical stress, prevent accumulation of mental stress, prevent accumulation of fatigue, and prevent immunosuppression, in a healthy human male or female having a sense of fatigue, in particular, a sense of fatigue caused by a mental load or a sense of fatigue caused by a mental load and a physical load, and can provide use, a method, and so on for these applications.

### Brief Description of Drawings

[Fig. 1] Figure 1 is a diagram illustrating the test items and assessment schedule.
[Fig. 2] Figure 2 shows graphs of results of OSA sleep analysis.
[Fig. 3] Figure 3 shows observed VAS (Visual Analog Scale) scores (means) of "Sense of fatigue" ("Sense of fatigue caused by mental load" or "Sense of fatigue caused by mental load and physical load") "before load application", "after mental load application", "after application of both loads", and "after rest period (after application of both loads)" at pre-ingestion (week 0) for all subjects.
[Fig. 4] Figure 4 is a table showing load-induced changes and ingestion-induced changes in VAS (Visual Analog Scale).
[Fig. 5] Figure 5 shows the results of VAS analysis with respect to the factors of "Clear-headedness", "Concentration", "Motivation", "Mood", "Frustration", and "Feeling of body heaviness".
[Fig. 6] Figure 6 is a table showing the results of POMS (Profile of Mood States) analysis.
[Fig. 7] Figure 7 shows changes in salivary cortisol concentration before and after mental load application after 4-week ingestion.
[Fig. 8] Figure 8 shows changes in salivary secretory immunoglobulin A (sIgA) concentration before test food ingestion and after 8-week ingestion.
[Fig. 9] Figure 9 shows temporal change and amount of change of oxidation markers at pre-ingestion (before load application) and after 8-week ingestion.
[Fig. 10] Figure 10 is a table showing data for subjects, body weights of subjects at pre-ingestion (before load application), after 4-week ingestion, after 8-week ingestion, and after 12-week ingestion, and blood (serum) astaxanthin (AX) levels in subjects after 4-week ingestion, after 8-week ingestion, and after 12-week ingestion.

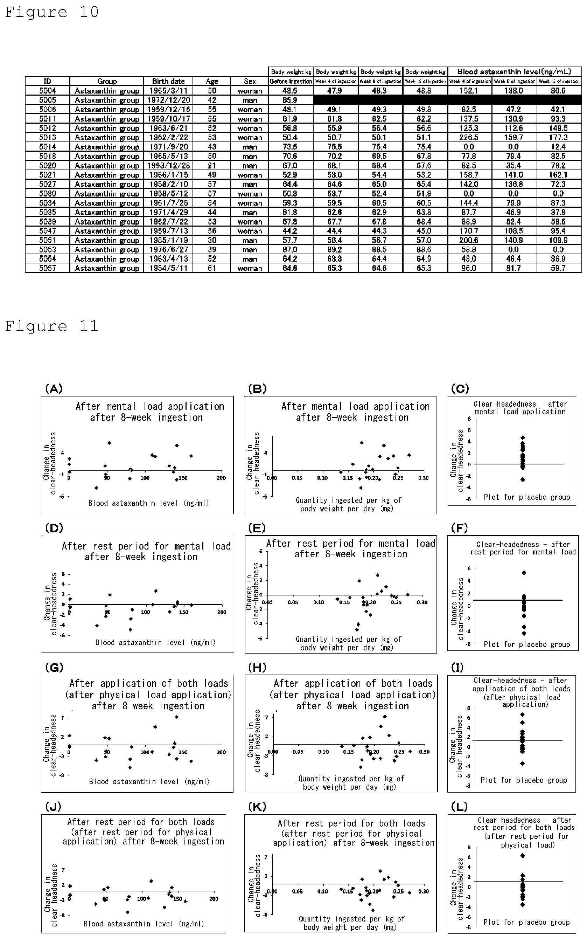

[Fig. 11] Figure 11 shows changes in VAS (plots for a placebo group; (C), (F), (I), and (L) in the figure) at each measurement point from before load application for the factor of "Clear-headedness" after 8-week ingestion; correlations between change in VAS at each measurement point from before load application for the same factor in an astaxanthin group and the blood (serum) astaxanthin level after 8-week ingestion ((A), (D), (G), and (J) in the figure); and correlations between change in VAS at each measurement point from before load application for the same factor in an astaxanthin group and ingestion of astaxanthin per kg of human body weight per day after 8-week ingestion ((B), (E), (H), and (K)).

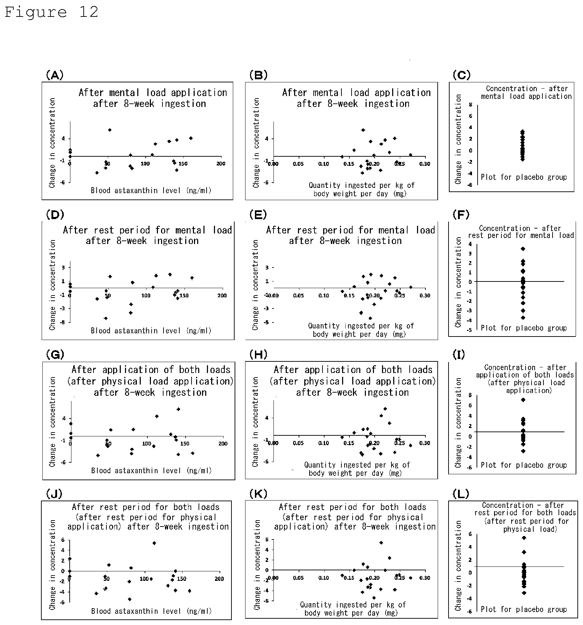

[Fig. 12] Figure 12 shows changes in VAS (plots for a placebo group; (C), (F), (I), and (L) in the figure) at each measurement point from before load application for the factor of "Concentration" after 8-week ingestion; correlations between change in VAS at each measurement point from before load application for the same factor in an astaxanthin group and the blood (serum) astaxanthin level after 8-week ingestion ((A), (D), (G), and (J) in the figure); and correlations between change in VAS at each measurement point from before load application for the same factor in an astaxanthin group and ingestion of astaxanthin per kg of human body weight per day after 8-week ingestion ((B), (E), (H), and (K) in the figure).

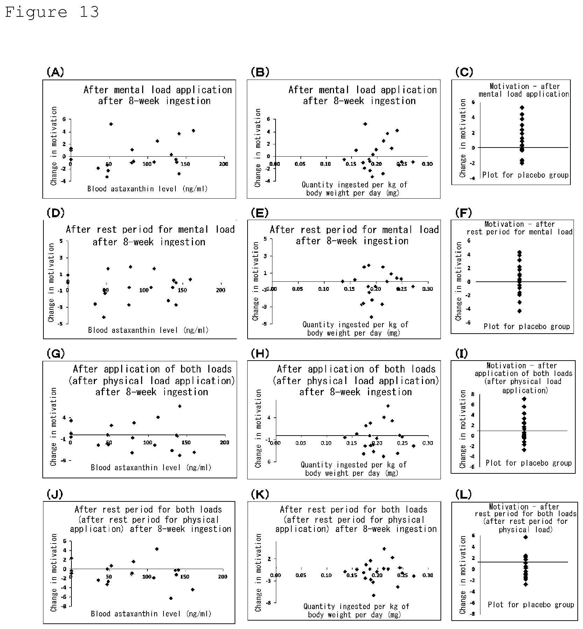

[Fig. 13] Figure 13 shows changes in VAS (plots for a placebo group; (C), (F), (I), and (L) in the figure) at each measurement point from before load application for the factor of "Motivation" after 8-week ingestion; correlations between change in VAS at each measurement point from before load application for the same factor in an astaxanthin group and the blood (serum) astaxanthin level after 8-week ingestion ((A), (D), (G), and (J) in the figure); and correlations between change in VAS at each measurement point from before load application for the same factor in an astaxanthin group and ingestion of astaxanthin per kg of human body weight per day after 8-week ingestion ((B), (E), (H), and (K) in the figure).

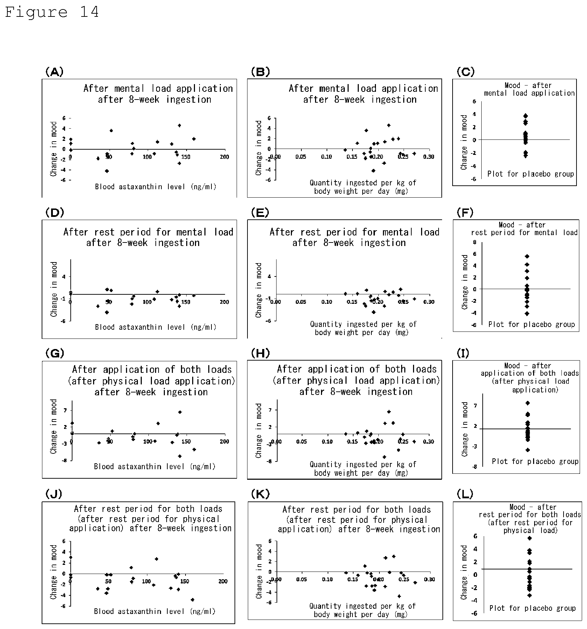

[Fig. 14] Figure 14 shows changes in VAS (plots for a placebo group; (C), (F), (I), and (L) in the figure) at each measurement point from before load application for the factor of "Mood" after 8-week ingestion; correlations between change in VAS at each measurement point from before load application for the same factor in an astaxanthin group and the blood (serum) astaxanthin level after 8-week ingestion ((A), (D), (G), and (J) in the figure); and correlations between change in VAS at each measurement point from before load application for the same factor in an astaxanthin group and ingestion of astaxanthin per kg of human body weight per day after 8-week ingestion ((B), (E), (H), and (K) in the figure).

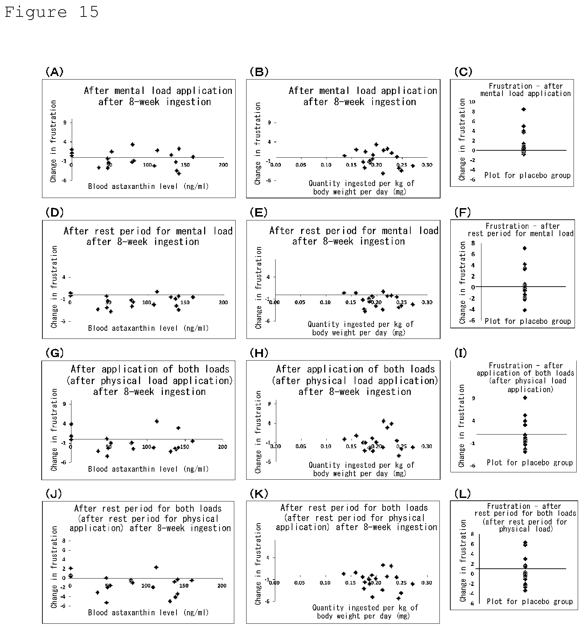

[Fig. 15] Figure 15 shows changes in VAS (plots for a placebo group; (C), (F), (I), and (L) in the figure) at each measurement point from before load application for the factor of "Frustration" after 8-week ingestion; correlations between change in VAS at each measurement point from before load application for the same factor in an astaxanthin group and the blood (serum) astaxanthin level after 8-week ingestion ((A), (D), (G), and (J) in the figure); and correlations between change in VAS at each measurement point from before load application for the same factor in an astaxanthin group and ingestion of astaxanthin per kg of human body weight per day after 8-week ingestion ((B), (E), (H), and (K) in the figure).

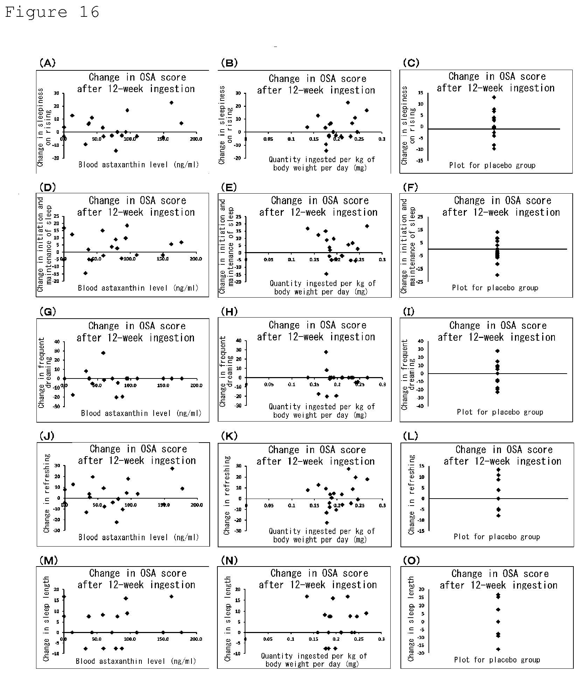

[Fig. 16] Figure 16 shows changes (plots for a placebo group; (C), (F), (I), (L), and (O) in the figure) from pre-ingestion (before load application) in the factors of "Sleepiness on rising", "Initiation and maintenance of sleep", "Frequent dreaming", "Refreshing", and "Sleep length" after 12-week ingestion; correlations between change from pre-ingestion (before load application) in each factor in an astaxanthin group and the blood (serum) astaxanthin level after 12-week ingestion ((A), (D), (G), (J), and (M) in the figure); and correlations between change from pre-ingestion (before load application) in each factor in an astaxanthin group and ingestion of astaxanthin per kg of human body weight per day after 12-week ingestion ((B), (E), (H), (K), and (N) in the figure).
[Fig. 17] Figure 17 is a table showing a relation between ingestion of astaxanthin per kg of human body weight per day measured after 8-week ingestion and load-induced change calculated on the basis of VAS (Visual Analog Scale) scores before load application and after a rest period for a mental load.
[Fig. 18] Figure 18 is a table showing results of a continuous calculation task to apply a mental load.
[Fig. 19] Figure 19 is a table showing results of a cycling task to apply a physical load.
[Fig. 20] Figure 20 shows a graph and table showing temporal change in the human blood (plasma) astaxanthin level when a food containing 60 mg of Haematococcus algae extract (6 mg as the free form of astaxanthin) and 270 mg of edible fat and oil per capsule was ingested at 2 capsules/day for 42 days, where (A) shows temporal change in the human blood (plasma) astaxanthin level and (B) shows all the data in the study.

### Description of Embodiments

Hereinafter, the food-and-drink composition, food-and-drink, pharmaceutical composition, or agent containing astaxanthin and/or astaxanthin-containing extract as an active ingredient according to the present invention will be described in detail. The food-and-drink composition, food-and-drink, pharmaceutical composition, or agent containing astaxanthin and/or astaxanthin-containing extract as an active ingredient according to the present invention can be differently applied to use or a method for these applications. The food-and-drink composition containing astaxanthin and/or astaxanthin-containing extract as an active ingredient according to the present invention is a food-and-drink composition containing astaxanthin and/or astaxanthin-containing extract as an active ingredient as one or more selected from the following (a), (b), (c), (d), (e), (f), and (g):
(a) a food-and-drink composition for improving dull-headedness, a food-and-drink for improving dull-headedness, a pharmaceutical composition for improving dull-headedness, or an agent for improving dull-headedness, for a human having a sense of fatigue;
(b) a food-and-drink composition for improving decreased concentration, a food-and-drink for improving decreased concentration, a pharmaceutical composition for improving decreased concentration, or an agent for improving decreased concentration, for a human having a sense of fatigue;
(c) a food-and-drink composition for improving decreased motivation, a food-and-drink for improving decreased motivation, a pharmaceutical composition for improving decreased motivation, or an agent for improving decreased motivation, for a human having a sense of fatigue;
(d) a food-and-drink composition for improving depressed mood, a food-and-drink for improving depressed mood, a pharmaceutical composition for improving depressed mood, or an agent for improving depressed mood, for a human having a sense of fatigue;
(e) a food-and-drink composition for resolving frustration, a food-and-drink for resolving frustration,
   a pharmaceutical composition for resolving frustration,
   or an agent for enhancing vigor, for a human having a sense of fatigue;
(f) a food-and-drink composition for reducing feeling of body heaviness, a food-and-drink for reducing feeling of body heaviness, a pharmaceutical composition for reducing feeling of body heaviness, or an agent for improving the quality of sleep, for a human having a sense of fatigue; and
(g) a food-and-drink composition for improving decreased vigor and/or activity, a food-and-drink for improving decreased vigor and/or activity, a pharmaceutical composition for improving decreased vigor and/or activity, or an agent for improving decreased vigor and/or activity, for a human having a sense of fatigue.

For the astaxanthin in the present invention, at least any one of natural astaxanthin and synthetic astaxanthin may be used, without any limitation. Examples of the natural astaxanthin include astaxanthins derived from algae including the genus Haematococcus; yeasts including the genus Phaffia; crustaceans such as shrimps, krill, and crabs; cephalopods such as squids and octopuses; various fish and shellfish; plants including the genus Adonis; bacteria including Paracoccus sp. N81106, Brevundimonas sp. SD212, and Erythrobacter sp. PC6; actinomycetes including Gordonia sp. KANMONKAZ-1129; Labyrintulea including Schizochytrium sp. KH105; and astaxanthin-producing genetically modified organisms. Preferred is astaxanthin extracted from microalgae including the genus Haematococcus, and more preferred is astaxanthin extracted from Haematococcus algae. Examples of the synthetic astaxanthin include AstaSana (DSM N.V.) and Lucantin Pink (R) (BASF SE). Examples of synthetic astaxanthin obtained by chemical conversion of another naturally-derived carotenoid include AstaMarine (PIVEG Inc.).

Examples of Haematococcus algae from which natural astaxanthin can be obtained include Haematococcus pluvialis, Haematococcus lacustris, Haematococcus capensis, Haematococcus deroebakensis, and Haematococcus zimbabwiensis.

To culture such Haematococcus green algae, a culture method with a sealed system is preferred, which prevents contamination with and proliferation of foreign microorganisms and reduces contamination with other contaminants. Examples of such culture methods include a method of culturing with a culture apparatus including a partially-open domed, conical, or cylindrical culture device and a gas jet unit freely movable within the device (International Publication No. WO 1999/050384); a method in which a drought stress is applied to Haematococcus algae to induce the algae to form cysts, and astaxanthin is collected from the culture of the algal cysts (Japanese Patent Laid-Open No. 1996-103288); a method of culturing through irradiation with light from a light source in the inside of a sealed culture apparatus; and a method with a sheet-shaped culture vessel or a tube-shaped culture vessel.

The astaxanthin and/or astaxanthin-containing extract applicable to the present invention may be, for example, astaxanthin and/or astaxanthin-containing extract obtained by crushing, as necessary, the cell walls of the above-described Haematococcus algae in accordance with a method disclosed in Japanese Patent Laid-Open No. 1993-068585 followed by extracting with an extraction medium or solvent such as an organic solvent such as acetone, ether, chloroform, and alcohol (e.g., ethanol, methanol) and supercritical carbon dioxide. In this case, the astaxanthin content of the astaxanthin-containing extract is preferably 3 to 40% (w/w), more preferably 3 to 12% (w/w), and even more preferably 5 to 10% (w/w).

Examples of the astaxanthin and/or astaxanthin-containing extract applicable to the present invention include commercially available products thereof. Examples of such commercially available products include ASTOTS series such as ASTOTS-S, ASTOTS-10O, ASTOTS-ECS, ASTPTS-2.0PW, and ASTOTS-3.0MB (ASTOTS is a registered trademark for all of the products; FUJIFILM Corporation); AstaReal, astavita, and astamate series such as AstaReal oil 50F, AstaReal oil 5F, AstaReal powder 20F, watersoluble AstaReal solution, AstaReal WS solution, AstaReal 10WS solution, AstaReal ACT, astavita e, astavita SPORTS, and astamate (registered trademark for all of the products; AstaReal Co., Ltd., Fuji Chemical Industries Co., Ltd.); BioAstin (registered trademark, Cyanotech Corporation); Astazine TM (BGG Japan); astaxanthin powder 1.5%, astaxanthin powder 2.5%, astaxanthin oil 5%, astaxanthin oil 10% (Bio Actives Japan Corporation); astaxanthin (Oryza Oil&Fat Chemical Co., Ltd.); SunActive AX (R) (Taiyo Kagaku Co., Ltd.); Haematococcus WS30 (YAEGAKI Bio-industry, Inc.); and AstaMarine (PIVEG Inc.).

The astaxanthin content of the composition of the present invention is based on the weight in terms of the free form of astaxanthin, and the astaxanthin content of the composition, for example, in the case of a food-and-drink, can be 0.000001 to 10% by weight, and preferably 0.00001 to 5% by weight, and in the case of a pharmaceutical product, can be 0.01 to 99% by weight, and preferably 0.1 to 90% by weight.

The composition of the present invention is suitably ingested for at least several days, preferably for one week, more preferably for four weeks, even more preferably for eight weeks, further preferably for 12 weeks, and the longer the period of ingestion is, the more the period is preferred. The frequency of ingestion per day may be one or more. The quantity of the composition of the present invention ingested can be set so that the quantity of astaxanthin ingested by an adult per day is 0.03 mg to 100 mg, preferably 0.05 mg to 30 mg, in terms of the free form of astaxanthin.

The term "sense of fatigue" in the present invention refers to a psychological factor which is a feeling associated with fatigue, languor, and listlessness and is also called malaise. The presence or absence of a "sense of fatigue" can be measured through VAS (Visual Analog Scale). More specifically, the VAS can be measured by instructing a subject to record the position corresponding to his/her feeling on a 10 cm line, as the far left end (0.0) on the line is defined as "best sensation with no fatigue", and the far right end (10.0) as "worst sensation of being too tired to do anything".

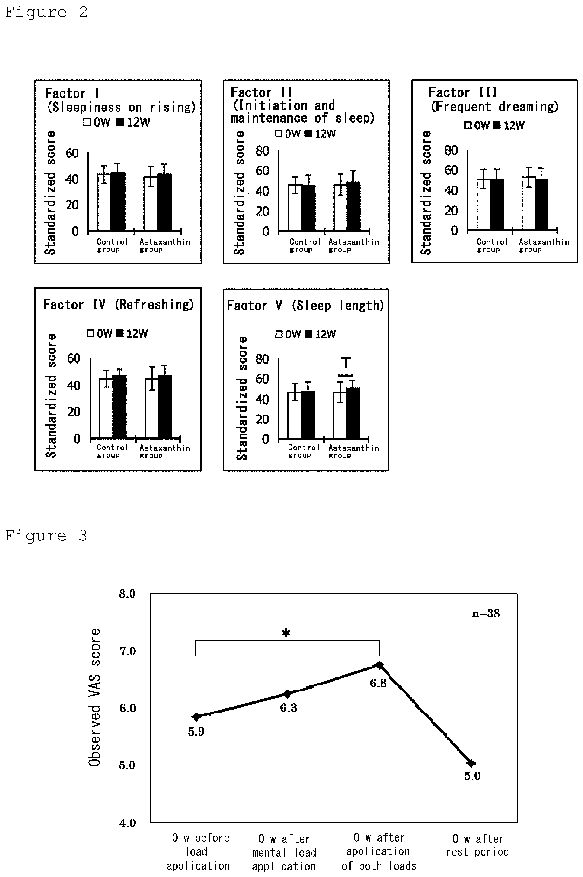

In Example in the present specification, a mental load or a mental load and a physical load are uniformly applied to all subjects, and the sense of fatigue felt by each subject is measured with VAS (Visual Analog Scale) analysis in terms of a sense of fatigue caused by a mental load and a sense of fatigue caused by a mental load and a physical load, the degree of improvement of dull-headedness, the degree of improvement of decreased concentration, the degree of improvement of decreased motivation, the degree of improvement of depressed mood, the degree of resolution of frustration, and the degree of reduction of feeling of body heaviness in a human, and measured with POMS (Profile of Mood Sates) analysis in terms of improvement of decreased vigor and/or activity in a human. Hence, the terms "metal fatigue" and "sense of fatigue caused by a mental load" are clearly discriminated herein, and, similarly, the terms "physical fatigue" and "sense of fatigue caused by a physical load" are clearly discriminated. Thus, the term "mental fatigue" is clearly different from the term "sense of fatigue caused by a mental load", and the term "physical fatigue" is clearly different from the term "sense of fatigue caused by a physical load". In the present specification, the term "sense of fatigue caused by a mental load" refers to, for example, the sense of fatigue at a point of "(0 w) after mental load application" in Figure 3, and the term "sense of fatigue caused by a mental load and a physical load" refers to, for example, the sense of fatigue at a point of "(0 w) after application of both loads" in Figure 3. The term "sense of fatigue caused by a mental load and a physical load" is clearly different from combination of "sense of physical fatigue" and "sense of mental fatigue", which are included in the term "sense of fatigue" as a total from (0 w) before load application to (0 w) after rest period in Figure 3, and derived by dividing the total into two parts.

Examples of the mental load include one or more loads selected from a load applied through a calculation task (calculation load), a load applied through a cognitive task (cognitive load), and a load applied through an information processing task (information processing task) to subjects. Examples of the calculation load include the Uchida-Kraepelin test; examples of the cognitive load include the Stroop Test (ST) and the Trail Making Test (TMT); and examples of the information processing load include the Wisconsin Card Sorting Test (WCST) and the Visual Display Terminal (VDT). Examples of the physical load include an exercise load such as an exercise performance test.

In the present invention, those skilled in the art can appropriately select from test items including interviews, mental loads (calculation loads; Uchida-Kraepelin test), physical loads (exercise loads; exercise performance test), blood tests, urinalyses, the OSA sleep analysis, the VAS (Visual Analog Scale), the POMS (Profile of Mood States), saliva sampling (measurement of salivary cortisol and salivary secretory immunoglobulin A (sIgA)), body weight measurement, and task performance evaluation, to evaluate and determine whether the effects to improve dull-headedness, improve decreased concentration, improve decreased motivation, improve depressed mood, resolve frustration, reduce feeling of body heaviness, improve decreased vigor and/or activity, enhance feeling of friendliness, improve or enhance the quality of sleep with respect to, for example, sleepiness on rising, initiation and maintenance of sleep, frequent dreaming, and sleep length, reduce, improve, or resolve physical stress, prevent accumulation of mental stress, prevent accumulation of fatigue, and prevent immunosuppression for a human having a sense of fatigue, in particular, a human having a sense of fatigue caused by a mental load or a sense of fatigue caused by a mental load and a physical load are exerted.

In the present invention, astaxanthin and/or astaxanthin-containing extract may exert, in a mode without any antioxidative effect, effects to improve dull-headedness, improve decreased concentration, improve decreased motivation, improve depressed mood, resolve frustration, reduce feeling of body heaviness, improve decreased vigor and/or activity, enhance feeling of friendliness, improve or enhance the quality of sleep with respect to, for example, sleepiness on rising, initiation and maintenance of sleep, frequent dreaming, and sleep length, reduce, improve, or resolve physical stress, prevent accumulation of mental stress, prevent accumulation of fatigue, and prevent immunosuppression for a human having a sense of fatigue, in particular, a human having a sense of fatigue caused by a mental load or a sense of fatigue caused by a mental load and a physical load.

The term "mode without any antioxidative effect" in the present invention refers to a mode in which antioxidative effect by astaxanthin and/or astaxanthin-containing extract is not exerted, or, in the case that astaxanthin and/or astaxanthin-containing extract is ingested together with a substance with antioxidant potential such as tocotrienol, refers to a mode in which antioxidative effect by astaxanthin and/or astaxanthin-containing extract and a substance with antioxidant potential such as tocotrienol is not exerted, and examples of such "mode without any antioxidative effect" include a human blood (serum) astaxanthin level; quantity of astaxanthin ingested per kg of human body weight per day; quantity of astaxanthin ingested per kg of human body weight per day and quantity of tocotrienol ingested per kg of human body weight per day; a combination of astaxanthin and/or astaxanthin-containing extract and a substance to be ingested therewith without any antioxidative effect; and the quantity ingested without any antioxidative effect per oxidative stress or antioxidant potential evaluated.

Regarding the "human blood (serum) astaxanthin level", at least one or more effects selected from the group of the effect to improve dull-headedness, the effect to improve decreased concentration, the effect to improve decreased motivation, the effect to improve depressed mood, the effect to resolve frustration, the effect to reduce feeling of body heaviness, the effect to improve decreased vigor and/or activity, the effect to enhance feeling of friendliness, the effect to improve or enhance the quality of sleep with respect to, for example, sleepiness on rising, initiation and maintenance of sleep, frequent dreaming, and sleep length, the effect to reduce, improve, or resolve physical stress, the effect to prevent accumulation of mental stress, the effect to prevent accumulation of fatigue, and the effect to prevent immunosuppression for a human having a sense of fatigue, in particular, a sense of fatigue caused by a mental load or a sense of fatigue caused by a mental load and a physical load are exerted when astaxanthin is ingested by (administered to) a subject in need thereof in a dosage achieving a human blood astaxanthin level equal to approximately 0 ng/mL to approximately 160 ng/mL, or approximately 0 ng/mL to approximately 150 ng/mL, or approximately 0 ng/mL to approximately 140 ng/mL, or approximately 0 ng/mL to approximately 135 ng/mL, or approximately 0 ng/mL to approximately 130 ng/mL, or approximately 0 ng/mL to approximately 120 ng/mL, or approximately 0 ng/mL to approximately 115 ng/mL, or approximately 0 ng/mL to approximately 110 ng/mL, or approximately 0 ng/mL to approximately 100 ng/mL, or approximately 0 ng/mL to approximately 90 ng/mL, or approximately 0 ng/mL to approximately 85 ng/mL, or approximately 0 ng/mL to approximately 80 ng/mL, or approximately 5 ng/mL to approximately 160 ng/mL, or approximately 5 ng/mL to approximately 150 ng/mL, or approximately 5 ng/mL to approximately 140 ng/mL, or approximately 5 ng/mL to approximately 135 ng/mL, or approximately 5 ng/mL to approximately 130 ng/mL, or approximately 5 ng/mL to approximately 120 ng/mL, or approximately 5 ng/mL to approximately 115 ng/mL, or approximately 5 ng/mL to approximately 110 ng/mL, or approximately 5 ng/mL to approximately 100 ng/mL, or approximately 5 ng/mL to approximately 90 ng/mL, or approximately 5 ng/mL to approximately 85 ng/mL, or approximately 5 ng/mL to approximately 80 ng/mL, or approximately 10 ng/mL to approximately 160 ng/mL, or approximately 10 ng/mL to approximately 150 ng/mL, or approximately 10 ng/mL to approximately 140 ng/mL, or approximately 10 ng/mL to approximately 135 ng/mL, or approximately 10 ng/mL to approximately 130 ng/mL, or approximately 10 ng/mL to approximately 120 ng/mL, or approximately 10 ng/mL to approximately 115 ng/mL, or approximately 10 ng/mL to approximately 110 ng/mL, or approximately 10 ng/mL to approximately 100 ng/mL, or approximately 10 ng/mL to approximately 90 ng/mL, or approximately 10 ng/mL to approximately 85 ng/mL, or approximately 10 ng/mL to approximately 80 ng/mL, or approximately 15 ng/mL to approximately 160 ng/mL, or approximately 15 ng/mL to approximately 150 ng/mL, or approximately 15 ng/mL to approximately 140 ng/mL, or approximately 15 ng/mL to approximately 135 ng/mL, or approximately 15 ng/mL to approximately 130 ng/mL, or approximately 15 ng/mL to approximately 120 ng/mL, or approximately 15 ng/mL to approximately 115 ng/mL, or approximately 15 ng/mL to approximately 110 ng/mL, or approximately 15 ng/mL to approximately 100 ng/mL, or approximately 15 ng/mL to approximately 90 ng/mL, or approximately 15 ng/mL to approximately 85 ng/mL, or approximately 15 ng/mL to approximately 80 ng/mL, or approximately 20 ng/mL to approximately 160 ng/mL, or approximately 20 ng/mL to approximately 150 ng/mL, or approximately 20 ng/mL to approximately 140 ng/mL, or approximately 20 ng/mL to approximately 135 ng/mL, or approximately 20 ng/mL to approximately 130 ng/mL, or approximately 20 ng/mL to approximately 120 ng/mL, or approximately 20 ng/mL to approximately 115 ng/mL, or approximately 20 ng/mL to approximately 110 ng/mL, or approximately 20 ng/mL to approximately 100 ng/mL, or approximately 20 ng/mL to approximately 90 ng/mL, or approximately 20 ng/mL to approximately 85 ng/mL, or approximately 20 ng/mL to approximately 80 ng/mL, or approximately 25 ng/mL to approximately 160 ng/mL, or approximately 25 ng/mL to approximately 150 ng/mL, or approximately 25 ng/mL to approximately 140 ng/mL, or approximately 25 ng/mL to approximately 135 ng/mL, or approximately 25 ng/mL to approximately 130 ng/mL, or approximately 25 ng/mL to approximately 120 ng/mL, or approximately 25 ng/mL to approximately 115 ng/mL, or approximately 25 ng/mL to approximately 110 ng/mL, or approximately 25 ng/mL to approximately 100 ng/mL, or approximately 25 ng/mL to approximately 90 ng/mL, or approximately 25 ng/mL to approximately 85 ng/mL, or approximately 25 ng/mL to approximately 80 ng/mL, or approximately 30 ng/mL to approximately 160 ng/mL, or approximately 30 ng/mL to approximately 150 ng/mL, or approximately 30 ng/mL to approximately 140 ng/mL, or approximately 30 ng/mL to approximately 135 ng/mL, or approximately 30 ng/mL to approximately 130 ng/mL, or approximately 30 ng/mL to approximately 120 ng/mL, or approximately 30 ng/mL to approximately 115 ng/mL, or approximately 30 ng/mL to approximately 110 ng/mL, or approximately 30 ng/mL to approximately 100 ng/mL, or approximately 30 ng/mL to approximately 90 ng/mL, or approximately 30 ng/mL to approximately 85 ng/mL, or approximately 30 ng/mL to approximately 80 ng/mL, or approximately 35 ng/mL to approximately 160 ng/mL, or approximately 35 ng/mL to approximately 150 ng/mL, or approximately 35 ng/mL to approximately 140 ng/mL, or approximately 35 ng/mL to approximately 135 ng/mL, or approximately 35 ng/mL to approximately 130 ng/mL, or approximately 35 ng/mL to approximately 120 ng/mL, or approximately 35 ng/mL to approximately 115 ng/mL, or approximately 35 ng/mL to approximately 110 ng/mL, or approximately 35 ng/mL to approximately 100 ng/mL, or approximately 35 ng/mL to approximately 90 ng/mL, or approximately 35 ng/mL to approximately 85 ng/mL, or approximately 35 ng/mL to approximately 80 ng/mL, or approximately 40 ng/mL to approximately 160 ng/mL, or approximately 40 ng/mL to approximately 150 ng/mL, or approximately 40 ng/mL to approximately 140 ng/mL, or approximately 40 ng/mL to approximately 135 ng/mL, or approximately 40 ng/mL to approximately 130 ng/mL, or approximately 40 ng/mL to approximately 120 ng/mL, or approximately 40 ng/mL to approximately 115 ng/mL, or approximately 40 ng/mL to approximately 110 ng/mL, or approximately 40 ng/mL to approximately 100 ng/mL, or approximately 40 ng/mL to approximately 90 ng/mL, or approximately 40 ng/mL to approximately 85 ng/mL, or approximately 40 ng/mL to approximately 80 ng/mL, or approximately 45 ng/mL to approximately 160 ng/mL, or approximately 45 ng/mL to approximately 150 ng/mL, or approximately 45 ng/mL to approximately 140 ng/mL, or approximately 45 ng/mL to approximately 135 ng/mL, or approximately 45 ng/mL to approximately 130 ng/mL, or approximately 45 ng/mL to approximately 120 ng/mL, or approximately 45 ng/mL to approximately 115 ng/mL, or approximately 45 ng/mL to approximately 110 ng/mL, or approximately 45 ng/mL to approximately 100 ng/mL, or approximately 45 ng/mL to approximately 90 ng/mL, or approximately 45 ng/mL to approximately 85 ng/mL, or approximately 45 ng/mL to approximately 80 ng/mL in terms of the free form of astaxanthin or an equivalent dosage thereof.

Here, the quantification limit for the blood (serum) astaxanthin level is 5 ng/mL in the present specification, and "approximately 0 ng/mL" refers to a level in the case that the blood (serum) astaxanthin is detected although it cannot be quantified. The term "approximately" in the phrases "approximately 5 ng/mL", "approximately 10 ng/mL", "approximately 15 ng/mL", "approximately 20 ng/mL", "approximately 25 ng/mL", "approximately 30 ng/mL", "approximately 35 ng/mL", "approximately 40 ng/mL", "approximately 45 ng/mL", "approximately 85 ng/mL", "approximately 90 ng/mL", "approximately 100 ng/mL", "approximately 110 ng/mL", "approximately 115 ng/mL", "approximately 120 ng/mL", "approximately 130 ng/mL", "approximately 135 ng/mL", "approximately 140 ng/mL", "approximately 150 ng/mL", and "approximately 160 ng/mL" is added because quantification results for the blood (serum) astaxanthin level vary to some degree.

To measure the blood (serum) astaxanthin level, a method which those skilled in the art can appropriately select can be used, and examples of such methods include, but are not limited to, a method in which a serum sample collected is purified and concentrated, and the resultant is subjected to reverse phase HPLC.

Regarding the "quantity of astaxanthin ingested per kg of human body weight per day", at least one or more effects selected from the group of the effect to improve dull-headedness, the effect to improve decreased concentration, the effect to improve decreased motivation, the effect to improve depressed mood, the effect to resolve frustration, the effect to reduce feeling of body heaviness, the effect to improve decreased vigor and/or activity, the effect to enhance feeling of friendliness, the effect to improve or enhance the quality of sleep with respect to, for example, sleepiness on rising, initiation and maintenance of sleep, frequent dreaming, and sleep length, the effect to reduce, improve, or resolve physical stress, the effect to prevent accumulation of mental stress, the effect to prevent accumulation of fatigue, and the effect to prevent immunosuppression for a human having a sense of fatigue, in particular, a sense of fatigue caused by a mental load or a sense of fatigue caused by a mental load and a physical load are exerted when astaxanthin is ingested by (administered to) a subject in need thereof in a dosage equal to approximately 0.1 mg to approximately 0.3 mg, or approximately 0.1 mg to approximately 0.275 mg, or approximately 0.1 mg to approximately 0.25 mg, or approximately 0.1 mg to approximately 0.225 mg, or approximately 0.1 mg to approximately 0.2 mg, or approximately 0.125 mg to approximately 0.3 mg, or approximately 0.125 mg to approximately 0.275 mg, or approximately 0.125 mg to approximately 0.25 mg, or approximately 0.125 mg to approximately 0.225 mg, or approximately 0.125 mg to approximately 0.2 mg, or approximately 0.15 mg to approximately 0.3 mg, or approximately 0.15 mg to approximately 0.275 mg, or approximately 0.15 mg to approximately 0.25 mg, or approximately 0.15 mg to approximately 0.225 mg, or approximately 0.15 mg to approximately 0.2 mg, or approximately 0.175 mg to approximately 0.3 mg, or approximately 0.175 mg to approximately 0.275 mg, or approximately 0.175 mg to approximately 0.25 mg, or approximately 0.175 mg to approximately 0.225 mg, or approximately 0.175 mg to approximately 0.2 mg, or approximately 0.2 mg to approximately 0.3 mg, or approximately 0.2 mg to approximately 0.275 mg, or approximately 0.2 mg to approximately 0.25 mg, or approximately 0.2 mg to approximately 0.225 mg per kg of body weight per day in terms of the free form of astaxanthin or an equivalent dosage thereof. Here, the term "approximately" in the phrases "approximately 0.1 mg", "approximately 0.125 mg", "approximately 0.15 mg", "approximately 0.175 mg", "approximately 0.2 mg", "approximately 0.225 mg", "approximately 0.25 mg", "approximately 0.275 mg", and "approximately 0.3 mg" is added because the quantity of astaxanthin ingested per kg of human body weight per day fluctuates to some degree.

The "combination of astaxanthin and/or astaxanthin-containing extract and a substance to be ingested therewith without any antioxidative effect" can be achieved through allowing the composition to further contain at least any of tocotrienol and olive oil.

In the case of a combination of tocotrienol and astaxanthin and/or astaxanthin-containing extract, at least one or more effects selected from the group of the effect to improve dull-headedness, the effect to improve decreased concentration, the effect to improve decreased motivation, the effect to improve depressed mood, the effect to resolve frustration, the effect to reduce feeling of body heaviness, the effect to improve decreased vigor and/or activity, the effect to enhance feeling of friendliness, the effect to improve or enhance the quality of sleep with respect to, for example, sleepiness on rising, initiation and maintenance of sleep, frequent dreaming, and sleep length, the effect to reduce, improve, or resolve physical stress, the effect to prevent accumulation of mental stress, the effect to prevent accumulation of fatigue, and the effect to prevent immunosuppression for a human having a sense of fatigue, in particular, a sense of fatigue caused by a mental load or a sense of fatigue caused by a mental load and a physical load are exerted when astaxanthin in a dosage equal to approximately 0.06 to approximately 0.14 mg per kg of human body weight per day in terms of the free form of astaxanthin or an equivalent dosage thereof and tocotrienol in a dosage equal to approximately 0.11 mg to approximately 0.23 mg per kg of human body weight per day or an equivalent dosage thereof are ingested by (administered to) a subject in need thereof.

Examples of the method for evaluating "oxidative stress or antioxidant potential" include, but are not limited to, a method of evaluation through measurement of the creatinine level, a method of evaluation through measurement of the oxidative stress level (Diacron-Reactive Oxygen Metabolites; d-ROMs), measurement of antioxidant potential (Biological Antioxidant Potential; BAP), a method of evaluation through calculation of the antioxidant potential (BAP) / oxidative stress (d-ROMs) ratio (GAP ratio), a method of evaluation through measurement of oxidative stress markers including the 8-hydroxydeoxyguanosine (8-OHdG) level, malondialdehyde level, oxidized LDL, oxidized RLP, NADPH oxidase, H₂O₂, and glutathione, and a method of evaluation through measurement of the oxidation stress index (OSI).

In the present specification, the term "mode without any antioxidative effect" can be interchangeable with the term "mode without any statistically significant antioxidative effect".

The composition of the present invention is a food-and-drink composition or a pharmaceutical composition, and the food-and-drink composition can be used to produce a food-and-drink such as a supplement, a solid food, a fluid food, and a beverage, for example, by using a method which those skilled in the art can appropriately select, and the pharmaceutical composition can be used to produce an agent such as a capsule, a solution, a suspension, an emulsion, a syrup, an elixir, an injection, a suppository, an inhalation, a nasal agent, and a transdermal agent, for example, by using a method which those skilled in the art can appropriately select.

For the effects to improve dull-headedness, improve decreased concentration, improve decreased motivation, improve depressed mood, resolve frustration, reduce feeling of body heaviness, improve decreased vigor and/or activity, enhance feeling of friendliness, improve or enhance the quality of sleep with respect to, for example, sleepiness on rising, initiation and maintenance of sleep, frequent dreaming, and sleep length, reduce, improve, or resolve physical stress, prevent accumulation of mental stress, prevent accumulation of fatigue, and prevent immunosuppression, for a human having a sense of fatigue, in particular, a sense of fatigue caused by a mental load or a sense of fatigue caused by a mental load and a physical load, the composition of the present invention can be ingested, for example, as a food for special dietary uses such as a food for specified health uses or a food with nutrient function claims, an infant formula, a food like a powdered milk for young children, a food like a powdered milk for lactating women, a food with health claims, a food for sick persons, a dairy product, or a fermented milk. In addition, the form may be any of a liquid, a paste, a powder, a solid, and so on, and the composition of the present invention can be blended in a food-and-drink to ingest as a food-and-drink. Examples of the food-and-drinks include milk, soft drinks, powder drinks, fermented milk, lactic acid bacteria drinks, acidic drinks, yogurt, cheese, bread, biscuits, crackers, pizza crusts, infant formulas, fluid foods, foods for sick persons, nutritional foods, frozen foods, food compositions, processed foods, and other commercially available foods. In the case that the composition of the present invention is used as a form of an acidic pharmaceutical product or food-and-drink, the pH can be set within 2.0 to 6.0, preferably within 3.0 to 5.0.

At least one nutrient selected from the group consisting of vitamins, peptides, minerals, organic acids or short-chain fatty acids, fatty acid esters, and organic bases can be additionally blended in the composition of the present invention. Alternatively, a fragrance, a sweetener, an acidulant, a colorant, etc., can be blended for the purpose of, for example, improvement of the taste or appearance. Tocotrienol or olive oil can be blended in the composition of the present invention, and, not only them, any of oils including sesame oil, rapeseed oil, safflower oil, and soybean oil can be blended.

Hereinafter, the food-and-drink composition, food-and-drink, pharmaceutical composition, and agent containing astaxanthin and/or astaxanthin-containing extract as an active ingredient, and use and a method for these applications, each according to the present invention, will be described on the basis of Example. The technical scope of the present invention is not limited to features illustrated in the Example.

### Example

### <Study 1>

### 1. Recruitment of subjects

Men and women aged between 20 and 64 years with a sense of fatigue who did not meet any of the criteria below were recruited as subjects.
- Chronic fatigue syndrome
- Previous history of diabetes, liver disease, kidney disease, gastrointestinal disease, peripheral vascular disorder, or other serious diseases
- Impaired cardiopulmonary function
- Abnormal liver/kidney function test results
- Previous history of gastrointestinal surgery
- Any disease currently under treatment
- Known allergy to any food or drug
- Individuals participating in intensive sport activities or currently dieting
- Intake of any health food, quasi-drugs, or pharmaceutical products (including OTC pharmaceutical products and prescription pharmaceutical products) containing any of the ingredients of the test food
- Consumption of an excessive amount of alcohol or inability to abstain from alcohol from the day before to the day of the study

Specifically, a total of 61 test candidates were asked to visit the clinic and undergo specified screening assessments, including an interview, physical examination, and laboratory tests. On the basis of the results, 39 individuals were recruited as the subjects. In the blood test included in the laboratory tests, measurement was performed for the total bilirubin level, AST (GOT) level, ALT (GPT) level, ALP level, LD (lactate dehydrogenase; LDH) level, γ-GT (γ-GTP) level, creatinine kinase (CK) level, total protein level, creatinine level, urea nitrogen level, uric acid level, total cholesterol level, TG (triglyceride) level, sodium level, potassium level, chloride level, calcium level, magnesium level, serum iron level, serum amylase level, HDL cholesterol level, LDL cholesterol level, ALB level (by a modified BCP method), white blood cell count, red blood cell count, hemoglobin level, hematocrit level, corpuscular constants (MCV, MCH, and MCHC), platelet count, blood glucose level, HbAlc level (NGSP), BAP level, hydroperoxide level (d-ROMs test), and blood (serum) astaxanthin level. In the urinalysis, measurement was performed for the creatinine level and 8-hydroxydeoxyguanosine (8-OHdG) level in combination with a qualitative test for protein, qualitative test for glucose, qualitative test for urobilinogen, qualitative test for bilirubin, specific gravity measurement, pH measurement, qualitative test for ketone bodies, and test for occult blood reaction. For the VAS (Visual Analog Scale), answers to Question 1 (sensation of fatigue) were examined. For the POMS (Profile of Mood States), TMD (Total Mood Disturbance) scores were calculated.

The investigator handed subjects informed consent form approved by the ethics committee before their entry into the study (before screening) and provided sufficient explanation of the following 12 items.
- The purpose, objective, duration, and procedure of the study
- Effects and anticipated adverse effects of the test food
- Adequate supervision of each subject by the investigator during the study period
- No disadvantages to the subjects if they did not consent to participate in the study
- Consent could be withdrawn at any time after providing consent initially
- Appropriate treatment would be available in the event of any hazard to health during the study
- Immediate conveyance of any new information that may affect subjects' willingness to continue participating in the study
- Protection of subjects' privacy including in the publication of study results
- Conditions that subjects must comply with
- Information about the study call center that subjects could contact to request more information about the study and their rights or in the event of a hazard to health potentially related to the study
- Disclosure of conflicts of interest
- Other matters
The investigator then provided the subjects with the opportunity to ask questions, answering them to each subject's satisfaction, and allowed them time to make a decision on study participation. The investigator obtained each subject's voluntary consent in writing.

### 2. Test foods

The compositions of the test foods are shown in Tables 1 and 2 below. A soft capsule (astavita-e; AstaReal Co., Ltd.) containing 120 mg (6 mg in terms of the free form of astaxanthin) of Haematococcus algae extract and 10 mg of tocotrienol mixture derived from palm oil was used as a test food for an astaxanthin group, and a control soft capsule containing 10 mg of tocotrienol mixture but containing no Haematococcus algae extract (astaxanthin) and being visually indistinguishable from the soft capsule as a test food for the astaxanthin group was used as a test food for a control group (placebo group).

**[Table 1]**

| [Test food for astaxanthin group] | |
|---|---|
| Name | estavita-e |
| Content | 330 mg/capsule |
| Haematococcus algae pigment (astaxanthin content) | 120 mg |
| | (6 mg) |
| Tocotrienol (total tocotrienol content: 45% or more) | 23 mg |
| Olive oil | 57 mg |
| Modified starch | 130 mg |
| Carrageenan(polysaccharide thickener) | |
| Glycerin | |
| Shape/package | shape: soft capsule/aluminum package |
| Storage conditions | Storing at room temperature without direct sunlight, high temperature and high humidity, and over loading avoided |
| Production date | 2015.08 |

**[Table 2]**

| [Test food for control group (placebo group)] | |
|---|---|
| Name | astavita-e placebo |
| Content | 330 mg/capsule |
| Tocotrienol | 23 mg |
| Olive oil | 177 mg |
| Modified starch | 130 mg |
| Carrageenan(polysaccharide thickener) | |
| Glycerin | |
| Shape/package | shape: soft capsule/aluminum package |
| Storage conditions | Storing at room temperature without direct sunlight, high temperature and high humidity, and overloading avoided |
| Production date | 2015.08 |

The test foods were each placed in an aluminum package. Personnel not directly involved in the study randomly provided each test food with an assignment number by using random numbers and distributed the test foods to the subjects, and thus a control group (placebo group) for ingestion of a capsule not containing astaxanthin and an astaxanthin group for ingestion of a capsule containing 6 mg of astaxanthin (120 mg of Haematococcus algae extract and 10 mg of tocotrienol mixture derived from palm oil) were established. The assignment list was sealed in an envelope by the personnel responsible for assignment and stored in a sealed state until taking out of the assignment list. After the subjects to be analyzed and data were fixed, the personnel responsible for assignment took out the assignment list to disclose the information.

### 3. Study period and site

Next, our company entrusted the study to Oneness Support (Osaka city, Osaka prefecture, Japan) via Fuji Chemical Industries Co., Ltd. (Nakaniikawa district, Toyama prefecture, Japan), and the study was conducted at Miura Clinic (Osaka city, Osaka prefecture, Japan) between August 2015 and December 2015. The study was performed in accordance with the study protocol and in strict compliance with the ethical principles of the Declaration of Helsinki (2013 revision) and the Ethical Guidelines for Medical Research in Humans (2015 revision), with approval by the Ethics Committee of Miura Clinic.

### 4. Ingestion of test foods

A placebo-controlled, randomized, double-blind, parallel-group study was employed for ingestion of the test food, and two groups, namely, the control group (placebo group) with ingestion of a capsule not containing astaxanthin and the astaxanthin group with ingestion of a capsule containing 6 mg of astaxanthin (120 mg of Haematococcus algae extract and 10 mg of tocotrienol mixture derived from palm oil) were compared through a double-blind method. For ingestion of the test food, the subjects were instructed to ingest one capsule twice daily (total 12 mg of astaxanthin (240 mg of Haematococcus algae extract and 20 mg of tocotrienol mixture derived from palm oil) in two capsules per day), one after breakfast (or after the first meal of the day if no breakfast was taken) and the other after dinner, with water or lukewarm water within 30 min of eating. The overall duration for ingestion was 8 weeks (12 weeks only for the OSA sleep analysis). The subjects were also instructed to record their test food ingestion, drinking, and exercise in a diary every day, from the first day to the last day of ingestion.

### 5. Test items and assessment schedule

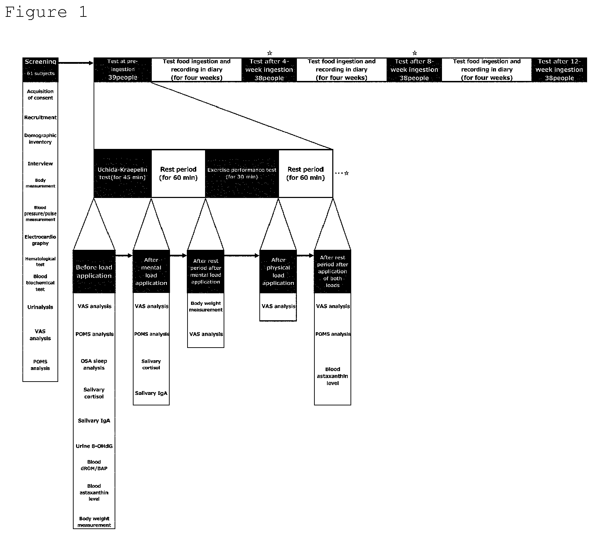

The test items and assessment schedule are illustrated in Figure 1. Recruitment of 39 subjects was as described in the above 1. After the recruitment, the 39 subjects were asked to visit the clinic at pre-ingestion (week 0, before load application) and undergo a mental load (calculation load; Uchida-Kraepelin test) and a physical load (exercise load; exercise performance test). Before and after load application, and after a rest period, the subjects participated in an interview, the OSA sleep analysis, VAS (Visual Analog Scale) analysis, POMS (Profile of Mood States) analysis, saliva sampling (measurement of salivary cortisol and salivary secretory immunoglobulin A (SIgA)), blood tests (measurement of the creatinine level, BAP level, hydroperoxide level (d-ROMs test), and blood (serum) astaxanthin level), urinalysis (measurement of the creatinine level and 8-hydroxydeoxyguanosine (8-OHdG) level), and body weight measurement. They then started ingestion of the test food, which they continued for 12 consecutive weeks. They were instructed to return to the clinic after 4-week, 8-week, and 12-week ingestion, where they underwent the same series of assessments excluding the POMS analysis, OSA sleep analysis, urinalysis, and some of the blood tests after 4-week ingestion, the same series of assessments excluding the OSA sleep analysis after 8-week ingestion, and only the OSA sleep analysis, body weight measurement, and some of the blood tests after 12-week ingestion.

### [5-1] Evaluation of subjective symptoms

OSA sleep analysis was performed before mental load application (calculation load, Uchida-Kraepelin test), and VAS analysis and POMS analysis were performed before and after mental load application and physical load application (exercise load; exercise performance test).

### (1) OSA sleep analysis

In accordance with a method reported in Yukari Yamamoto, Hideki Tanaka, Miki Takase, Katuo Yamazaki, Kazuo Azumi, and Shuichiro Shirakawa: "Development and standardization of OSA sleep inventory (MA version) for middle-aged and elderly individuals", Brain Science and Mental Disorders, vol. 10, p401-409, 1999, the subjects were instructed to evaluate with respect to items listed in the OSA sleep inventory, and the results were analyzed. The assessment was performed once in the morning of the day of assessment (before load application). The OSA sleep inventory (MA version) consists of 16 items in terms of five factors, namely, First factor: sleepiness on rising, Second factor: initiation and maintenance of sleep, Third factor: frequent dreaming, Fourth factor: refreshing, and Fifth factor: sleep length, and the subjects were instructed to answer each question from four choices, and the results were converted to scores with a conversion table for analysis.

### (2) VAS (Visual Analog Scale)

The assessment to the subjects consisted of seven questions: "Sense of Fatigue ("sense of fatigue caused by mental load" or "sense of fatigue caused by mental load and physical load") (best sensation with no fatigue - worst sensation of being too tired to do anything)", "Clear-headedness (very clear - very dull)", "Concentration (able to concentrate very easily - unable to concentrate at all)", "Motivation (highly motivated - totally unmotivated)", "Mood (very happy - as depressed as I can be)", "Frustration (not frustrated at all - very frustrated)", and "Feeling of body heaviness (feeling my body is very light - feeling my body is very heavy)". For each question, the subjects were instructed to draw a vertical line by intuition on a 100 mm horizontal line to indicate their condition at the time, with the far left end indicating the best condition and the far right end indicating the worst condition. A VAS score was derived by measuring with a ruler the distance from the far left end to the intersection with the vertical line. VAS analysis was performed five times in total in one day: in the morning of the day of assessment (before load application), after application of a physical load and a mental load, at the end of the assessment visit (after a rest period), after mental load application (calculation load; Uchida-Kraepelin test), and after a rest period after mental load application. To determine transient changes in fatigue, the load-induced change was calculated by subtracting the pre-load value from the values after mental load application, after a rest period after mental load application, after application of both loads, and after a rest period therefor. In addition, to determine the effect of test food ingestion alone, ingestion-induced change was calculated by subtracting the pre-load (pre-ingestion) value at week 0 from the pre-load value after 4-week ingestion and after 8-week ingestion.

### (3) POMS (Profile of Mood States)

In order to quantify the degree of quality of life (QOL) or stress, various evaluation tests to evaluate emotion (feeling/mood) such as anxiety and tension have been developed. Among them, the POMS is very useful for total evaluation of almost all of the feelings or moods elicited as a result of stress response.

The POMS, which was developed by McNair et al. (1971), is an evaluation test enabling simultaneous measurement of temporal feeling or mood and composed of seven scales, namely, anger-hostility: AH, confusion-bewilderment: CB, depression-dejection: DD, fatigue-inertia: FI, tension-anxiety: TA, vigor-activity: VA, and friendliness: F, through 65 questions. Among the seven scales, anger-hostility (AH), confusion-bewilderment (CB), depression-dejection (DD), fatigue-inertia (FI), and tension-anxiety (TA), are negative feeling factors, and vigor-activity (VA) and friendliness (F) are positive feeling factors. The POMS is used for body and mind check and conditioning for athletes, and condition check for mood and degree of fatigue and environmental improvement in the field of nursing care and welfare, and has been demonstrated to be highly reliable and very sensitive scales for assessment of effects for various experimental studies.

Using the self-administered POMS 2 in Japanese, adult short version (Kanekoshobo, Tokyo, Japan), the subjects answered 35 questions by rating their "current mood" on a 5-point scale (0 = not at all; 1 = a little; 2 = moderately; 3 = quite a bit; 4 = extremely). Responses to each question were scored according to the POMS scoring system for the seven scales: anger-hostility, confusion-bewilderment, depression-dejection, fatigue-inertia, tension-anxiety, vigor-activity, and friendliness, and the raw scores were converted into TMD scores with a conversion table. The POMS was administered 3 times in total in one day: in the morning of the day of assessment (before load application), after mental load application (calculation load; Uchida-Kraepelin test), and at the end of the day of assessment (after the rest period). To determine transient changes in fatigue, the load-induced change was calculated by subtracting the pre-load value from the values after mental load application and after a rest period therefor. In addition, to determine the effect of test food ingestion alone, ingestion-induced change was calculated by subtracting the pre-load (pre-ingestion) value at week 0 from the pre-load value after 8-week ingestion.

### [5-2] Measurement of salivary cortisol and salivary secretory immunoglobulin A (sIgA)

Salivary cortisol, which was examined as a biochemical index for a mental load, is used as a stress marker because of simplicity of measurement due to the high correlation with blood cortisol, and known to be increased by an acute mental stress, for example, caused at making a speech in front of others or doing mental calculation or by continuous, high-intensity exercise. Because the salivary cortisol concentration is known to show circadian variation, being high in the morning and then declining as the night approaches, mental load application (calculation load; Uchida-Kraepelin test) was performed in the morning in this study. Salivary secretory immunoglobulin A (sIgA) is an antibody produced by B cells that inhibits the proliferation of pathogens on the mucosa of the oral cavity, airway, intestines, and other organs. It is known that reduction of sIgA in saliva is associated with the development of upper respiratory tract infection and that salivary sIgA concentration decreases under chronic stress and increases in a relaxed state.

### (1) Measurement of salivary cortisol

Prior to saliva sampling, the subjects were instructed to brush their teeth and place a cotton swab (Salivette) in the mouth for 2 minutes without chewing to thoroughly soak the cotton swab with saliva. Saliva samples were collected twice, in the morning of the day of assessment (before load application) and after mental load application (calculation load; Uchida-Kraepelin test). Salivary cortisol concentration was measured with a competitive chemiluminescent immunoassay (CLIA) kit (Chemilumi ACS-E Cortisol II; Siemens Healthcare Diagnostics Inc.).

### (2) Measurement of salivary secretory immunoglobulin A (sIgA)

Saliva samples were collected from the subjects in the morning of the day of assessment (before load application) in the same manner as in (1) of [5-2]. The sIgA concentration in each saliva sample was measured with an enzyme immunoassay (EIA) kit (EIA s-IgA Test Kit, Medical & Biological Laboratories Co., Ltd.).

### [5-3] Mental and physical loads and evaluation of task performance

Mental load application (calculation load; Uchida-Kraepelin test) was followed by a 60-min rest period and then by physical load application (exercise load; exercise performance test).

### (1) Mental load (calculation load; Uchida-Kraepelin test)

A mental load was applied to each subject by a continuous calculation task with the Uchida-Kraepelin test (Nisseiken Inc., Japan) consisting of two sessions of a 15-min serial addition task (total 30 min), with a 5-min rest period between the first and second sessions. Specifically, the subjects were instructed to sequentially add two adjoining single-digit numbers (combination of 3, 4, 5, 6, 7, 8, and 9), record only the last digit (one's place) of the sum, spend only 1 min on each line, and then quickly move on to the next line of numbers for calculation. This task was continued for 15 min in each session. After the completion of the task, the number of calculations and the number of wrong answers were counted to calculate the percentage of correct answers, and the results for the first session were subtracted from those for the second session to determine change in task performance in the second session, which was used as the task performance with a mental load.

### (2) Physical load (exercise load; exercise performance test)

A physical load was applied by a cycling task on a bicycle ergometer. Specifically, the subjects were instructed to pedal for 30 min with a rotation rate of the pedal adjusted to maintain the heart rate at 120 to 130 HR for men and 110 to 120 HR for women. The magnitude of the load was set at a constant level of 80 W for men and 70 W for women. The distance cycled (km) in 30 min was measured, which was used as the task performance with a physical load.

### [5-4] Measurement of blood (serum) astaxanthin level (free form of astaxanthin) (trans-form)

Measurement of the blood (serum) astaxanthin level was performed for each subject of each group after 4-week ingestion, after 8-week ingestion, and after 12-week ingestion. Here, the limit of measurement was 5 ng/mL, and thus the blood (serum) astaxanthin level in the case that the measurement was lower than the limit of measurement was regarded as approximately 0 ng/mL in the following.

### (1) Treatment of samples

To 100 µL of a serum or plasma sample collected from each subject, 500 µL of ethanol solution of butylhydroxytoluene (BHT; 50 µg/mL), 100 µL of 100 ng/mL acetone solution of an internal standard (ethyl 8'-apo-beta-caroten-8'-oate, Carote Nature, 1010), and 500 µL of distilled water were added, and the resultant was vigorously stirred for 15 seconds with a vortex mixer, and then 5 mL of hexane was added thereto and the resultant was further vigorously stirred for 15 seconds with a vortex mixer. After this stirring process was repeated three times, centrifugation was performed at a rotational frequency of 3500 rpm for 10 minutes. After the centrifugation, 4 mL of the supernatant was collected and filtered through a membrane filter with a mesh size of 0.45 µm. The resulting filtrate was concentrated with a centrifugal evaporator, and then redissolved in 150 µL of acetone, and subjected to reverse phase HPLC. Separately, standard solution prepared by combining 100 mL of 100 ng/mL acetone solution of an astaxanthin standard reagent (Astaxanthin, ALEXIS BIOCHEMICALS, 460-031-M250) and 1 mL of 10 µg/mL acetone solution of an internal standard (ethyl 8'-apo-beta-caroten-8'-oate) was subjected to reverse phase HPLC in the same manner, and the astaxanthin level was determined through comparison of the peak area ratio obtained.

### (2) HPLC conditions

For the HPLC and analysis column, Shimadzu LC20A series (pump: LC-20AD, degasser: DGU-20A5R, autosampler: SIL-20AC, column oven: CTO-20AC, detector: SPD-20AV, system controller: CBM-20A) and a YMC-Carotenoid (4.6 × 250 mm, particle diameter: 5 µm) were used, respectively. For the mobile phase, Solution A (methanol), Solution B (tert-butyl methyl ether), and Solution C (1% aqueous solution of phosphoric acid) were used, and gradient elution was performed so that the mixing ratio of Solution A to Solution B was 93:5 (%ratio) at initiation and the fraction of Solution B reached 16% at minute 4, 22.5% at minute 7, 48.75% at minute 25.6, and 90% at minute 33.2, and the fraction of Solution B was then retained at 90% until minute 41.5, and the mixing ratio was returned to the initial mixing ratio at minute 41.7, and elution was performed at the initial mixing ratio until minute 53.4. The fraction of Solution C was set at a constant fraction of 2%, the temperature of the column oven was set at 16°C, and measurement was performed by using the UV/VIS detector at a detection wavelength of 470 nm with a flow rate of 1 mL/min.

### [5-5] Other measurements

Blood tests (BAP level, hydroperoxide level (d-ROMs test)), urinalysis (measurement of creatinine level and 8-hydroxydeoxyguanosine (8-OHdG) level), and body weight measurement were performed. The blood tests were performed before mental load application (calculation load; Uchida-Kraepelin test) and after physical load application (exercise load; exercise performance test) at pre-ingestion (before load application), and at least any of week 4 of ingestion, week 8 of ingestion, and week 12 of ingestion. The urinalysis was performed before mental load application at pre-ingestion (before load application) and after 8-week ingestion. The body weight measurement was performed before and after mental load application at pre-ingestion (before load application), after 4-week ingestion, after 8-week ingestion, and after 12-week ingestion.

### 6. Rest period and food intake on days of assessment

The subjects were asked to eat two rice balls and drink 100 mL of water within 10 min of the beginning of the rest period after mental load application (calculation load; Uchida-Kraepelin test) and to drink 100 mL of water during the 60-min rest period after physical load application (exercise load; exercise performance test).

### 7. Statistical Analysis

Intergroup comparison was made for VAS (Visual Analog Scale) scores, POMS (Profile of Mood States) scores, and task performance between the control (placebo) and astaxanthin groups using Student's t-test. Intragroup comparison was made for the OSA, salivary cortisol concentration, salivary sIgA concentration, creatinine level, BAP level, d-ROMs level, d-ROMs/BAP ratio, and 8-OHdG level by using the paired t-test. Results were expressed as mean and standard error (SE). Differences were considered significant at two-tailed significance levels of less than 5% and marginally significant at two-tailed significance levels of 5% or more and less than 10%, respectively. Means and standard deviations were calculated for the creatinine level, BAP level, d-ROMs level, d-ROMs/BAP ratio, 8-OHdG level, and blood (serum) astaxanthin level.

### <Study 2>

This study is a study in humans to confirm how the blood (plasma) astaxanthin level changes through long-term ingestion of astaxanthin. While the blood (serum) astaxanthin level was measured at the completion of ingestion (after 12-week ingestion) in Study 1, Study 2 is, specifically, a study in humans to confirm how the blood (plasma) astaxanthin level behaves in the case that the period of ingestion is shorter than 12 weeks.

### 1. Subjects

Ten healthy Japanese men and women at age of 20 years or older and younger than 50 years

### 2. Test foods and ingestion

Each subject ingested two capsules per day, each capsule containing 60 mg (6 mg in terms of the free form of astaxanthin) of Haematococcus algae extract and 270 mg of edible fat and oil, with water 30 minutes after breakfast (12 mg/day in terms of the free form of astaxanthin) for 42 days.

### 3. Blood sampling and quantification of astaxanthin

Blood sampling was performed one day, six days, 13 days, 20 days, 27 days, 34 days, and 42 days after the initiation of ingestion of the capsules to obtain the plasma. The plasma astaxanthin level was measured by using the method in [5-4] in Study 1 with HPLC.

### <Results 1>

### 1. Subject demographics

Nineteen subjects (8 men, 11 women) were assigned to the control group (placebo group), and 20 subjects (9 men, 11 women) to the astaxanthin group. Mean age for the control group was 48.2 (range: 25 to 64) years and 48.7 (range: 21 to 61) years for the astaxanthin group, showing no significant difference between them. After entry to the study, one subject in the astaxanthin group was withdrawn from the study, and the total number of subjects in the astaxanthin group reduced to 19. After exclusion of this subject, the mean age in the astaxanthin group was 49.0 years, with no significant difference in comparison with that for the control group. The compliance rates for test food ingestion in the control group and the astaxanthin group were 96.5% and 98.1%, respectively, again showing no significant difference between the groups. None of the subjects met any of the exclusion criteria; hence all of the subjects were included in the analysis.

### 2. Evaluation of subjective symptoms

### [2-1] OSA sleep analysis

The results of OSA sleep analysis are shown in Figure 2. As shown in Figure 2, among the five factors, namely, First factor (sleepiness on rising), Second factor (initiation and maintenance of sleep), Third factor (frequent dreaming), Fourth factor (refreshing), and Fifth factor (sleep length), marginally significant increase was observed for the astaxanthin group with respect to Fifth factor (sleep length) in comparison with that at pre-ingestion (before load application). Although no significant difference was found, a large increase was observed for the astaxanthin group with respect to First factor (sleepiness on rising), Second factor (initiation and maintenance of sleep), and Fourth factor (refreshing) in comparison with those at pre-ingestion (before load application).

As is clear from these results, this study revealed that the astaxanthin and/or astaxanthin-containing extract exerts not the conventionally-known action and effect to improve sleep disorders, but the effect to improve or enhance the quality of sleep with respect to, for example, sleepiness on rising, initiation and maintenance of sleep, frequent dreaming, and sleep length in a healthy human having a sense of fatigue, in particular, a sense of fatigue caused by a mental load (calculation load) or a sense of fatigue caused by a mental load (calculation load) and a physical load (exercise load).

The results of this study showed that the degree of improvement in Fifth factor (sleep length) was larger than that when 200 mL of chamomile tea is taken approximately 1 hour before bedtime over three days (Sadako Sato et al., Articles of the 42nd (2011) Japanese Nursing Association Annual General Convention, Adult Nursing II, 2012, p. 80-83).

### [2-2] VAS (Visual Analog Scale)

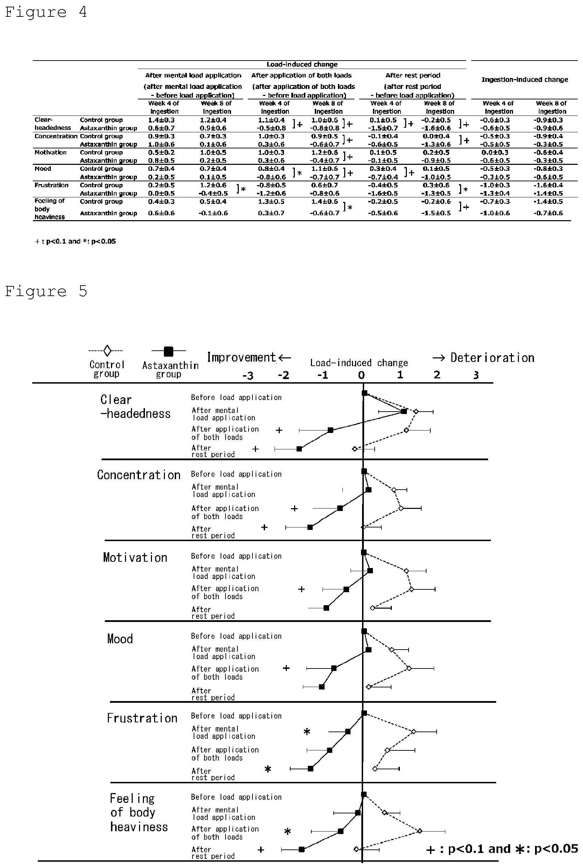

The results of VAS analysis are shown in Figures 3, 4, and 5. Figure 3 demonstrates that, after mental load application at pre-ingestion (week 0), when the presence or absence of a sense of fatigue caused by a mental load can be determined, the mean of VAS scores of all the subjects indicated a sense of fatigue caused by a mental load, and that, after application of both loads at pre-ingestion (week 0), when the presence or absence of a sense of fatigue caused by a mental load and a physical load can be determined, the mean of VAS scores of all the subjects indicated a sense of fatigue caused by a mental load and a physical load, and, in addition, it was significantly larger than that before load application at pre-ingestion (week 0). Thus, it can be seen that the mean of VAS scores of all the subjects after application of both loads at pre-ingestion (week 0) notably indicated a sense of fatigue caused by a mental load and a physical load. While these results each show load-induced change to determine transient changes in fatigue, Figure 4 demonstrates that the astaxanthin group for the factor of "Mood" exhibited significant improvement after application of both loads after 4-week ingestion in comparison with the control group, and exhibited marginally significant improvement after application of both loads after 8-week ingestion and after the rest period (after application of both loads) after 4-week ingestion in comparison with the control group; the astaxanthin group for the factor of "Frustration" relating to the sense of mental fatigue exhibited significant improvement after mental load application and after the rest period (after application of both loads) after 8-week ingestion in comparison with the control group; the astaxanthin group for the factor of "Feeling of body heaviness" exhibited significant improvement after application of both loads after 8-week ingestion in comparison with the control group, and exhibited marginally significant improvement after the rest period (after application of both loads) after 8-week ingestion in comparison with the control group; the astaxanthin group for the factor of "Clear-headedness" exhibited marginally significant improvement and enhancement after application of both loads and after the rest period (after application of both loads) after 4-week ingestion and after 8-week ingestion in comparison with the control group; the astaxanthin group for the factor of "Concentration" exhibited marginally significant improvement and enhancement after application of both loads and after the rest period (after application of both loads) after 8-week ingestion in comparison with the control group; and the astaxanthin group for the factor of "Motivation" exhibited marginally significant enhancement after application of both loads after 8-week ingestion in comparison with the control group. Further, Figure 5 demonstrates that the astaxanthin group for the factor of "Clear-headedness" or "Concentration" exhibited improvement of decreased clear-headedness or decreased concentration after mental load application, after application of both loads, and after the rest period (after application of both loads), in particular, exhibited marginally significant improvement after application of both loads and after the rest period (after application of both loads), in comparison with the control group; the astaxanthin group for the factor of "Motivation" and the factor of "Mood" exhibited improvement of decreased motivation or depressed mood after mental load application, after application of both loads, and after the rest period (after application of both loads), in particular, exhibited marginally significant improvement after application of both loads, in comparison with the control group; the astaxanthin group for the factor of "Frustration" exhibited resolution of frustration (improvement of deteriorated frustration) after mental load application, after application of both loads, and after the rest period (after application of both loads), in particular, exhibited significant improvement after mental load application and after the rest period (after application of both loads), in comparison with the control group; and the astaxanthin group for the factor of "Feeling of body heaviness" exhibited reduction of feeling of body heaviness (improvement of deteriorated feeling of body heaviness) after mental load application, after application of both loads, and after the rest period (after application of both loads), in particular, exhibited significant improvement after application of both loads and exhibited marginally significant improvement after the rest period (after application of both loads), in comparison with the control group.

These results revealed that the astaxanthin and/or astaxanthin-containing extract exerts effects to improve dull-headedness, improve decreased concentration, improve decreased motivation, improve depressed mood, resolve frustration (improve deteriorated frustration), and reduce feeling of body heaviness (improve deteriorated feeling of body heaviness), in a healthy human having a sense of fatigue, in particular, a sense of fatigue caused by a mental load or a sense of fatigue caused by a mental load and a physical load.

### [2-3] POMS (Profile of Mood States)

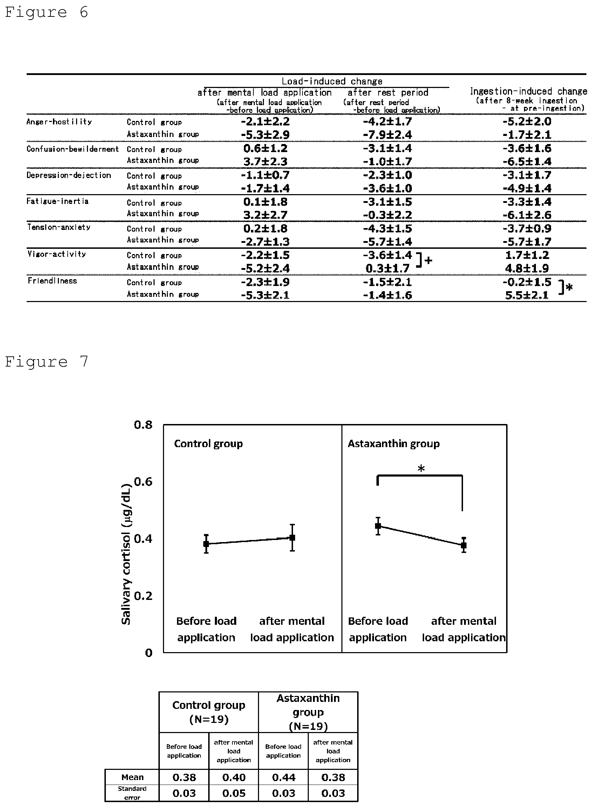

Figure 6 shows the results of POMS analysis. These results each show load-induced change to determine transient changes in fatigue. Figure 6 demonstrates that, with respect to load-induced change to determine transient changes in fatigue, the astaxanthin group for the factor of "Vigor-activity (VA)" exhibited a marginally significant high value after the rest period after 8-week ingestion in comparison with the control group; with respect to ingestion-induced change (after 8-week ingestion - at pre-ingestion (before load application)) to determine the effect of test food ingestion alone, the astaxanthin group for the factor of "Friendliness (F)" exhibited a significant high value in comparison with the control group.

These results revealed that the astaxanthin and/or astaxanthin-containing extract exerts effects to improve decreased vigor and/or activity in a healthy human having a sense of fatigue, in particular, a sense of fatigue caused by a mental load or a sense of fatigue caused by a mental load and a physical load, and enhance or increase feeling of friendliness in a healthy human having a sense of fatigue, in particular, a sense of fatigue caused by a mental load or a sense of fatigue caused by a mental load and a physical load.

The results of this study showed that the improvement in the factor of "Vigor-activity (VA)" and other factors (factors of "Anger-hostility (AH)", "Fatigue-inertia (FI)", and "Confusion-bewilderment (CB)") was larger than that when bonito stock is taken in the morning and evening over two weeks (Taichi Ishizaki et al., Journal of the Japanese Society for Food Science and Technology, Vol. 53 (4), April 2006, p. 225-228).

In addition, the results of this study showed that the improvement in the factor of "Vigor-activity (VA)" was larger than that when 250 mg of quercetin and 100 mg of isoquercetin in total are ingested in two administrations in the morning and evening for 42 to 54 days (Kevin A. et al., MILITARY MEDICINE, Vol. 176 (5), May 2011, p. 565-572), and the improvement when 250 mg of quercetin and 100 mg of isoquercetin are ingested was a negative value (rather deteriorated).

### 3. Evaluation of biochemical indices

### [3-1] Salivary cortisol

Figure 7 shows change in salivary cortisol concentration before and after mental load application after 4-week ingestion. As can be seen from Figure 7, after 4-week ingestion, the salivary cortisol concentration after mental load application in the astaxanthin group was significantly lower than the salivary cortisol concentration before mental load application; however, no significant change was found between the salivary cortisol concentration before mental load application and that after mental load application in the control group (placebo group). With respect to salivary cortisol concentration (µg/dL) before and after mental load application after 8-week ingestion, the values before and after mental load application in the control group were 0.38 ± 0.03 and 0.40 ± 0.05, respectively. On the other hand, the values before and after mental load application in the astaxanthin group were 0.44 ± 0.03 and 0.38 ± 0.03, respectively.

From these results, the decrease of the salivary cortisol in the astaxanthin group objectively demonstrated that a physical stress was reduced in a healthy human having a sense of fatigue, in particular, a sense of fatigue caused by a mental load or a sense of fatigue caused by a mental load and a physical load, and revealed that the astaxanthin and/or astaxanthin-containing extract exerts effects to reduce, improve, or resolve a physical stress in a healthy human having a sense of fatigue, in particular, a sense of fatigue caused by a mental load or a sense of fatigue caused by a mental load and a physical load.

### [3-2] Salivary secretary immunoglobulin A (sIgA)

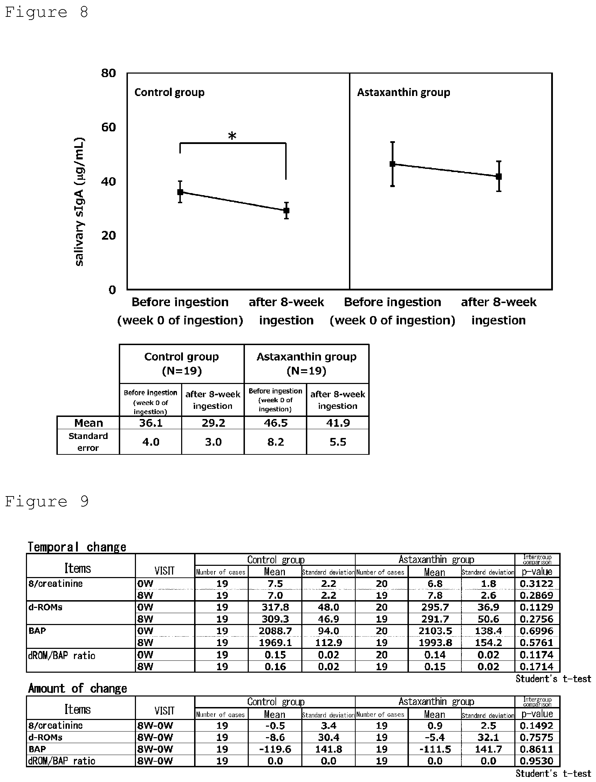

Figure 8 shows changes in salivary secretary immunoglobulin A (sIgA) concentration before test food ingestion and after 8-week ingestion. As can be seen from Figure 8, the salivary sIgA concentration after 8-week ingestion in the control group (placebo group) was significantly lower than the salivary sIgA concentration at pre-ingestion (before load application); however, no significant change was found for salivary sIgA concentration in the astaxanthin group until week 8 of ingestion. With respect to salivary sIgA concentration (µg/mL), the values at pre-ingestion (before load application) and after 8-week ingestion in the control group were 36.1 ± 4.0 and 29.2 ± 3.0, respectively. On the other hand, the values at pre-ingestion (before load application) and after 8-week ingestion in the astaxanthin group were 46.5 ± 8.2 and 41.9 ± 5.5, respectively.

Accumulation of mental stress or fatigue leads to vulnerability to a cold or herpes labialis. The above results suggested that ingestion of astaxanthin can prevent accumulation of mental stress, accumulation of fatigue, and immunosuppression. That is, it was revealed that the astaxanthin and/or astaxanthin-containing extract exerts effects to prevent accumulation of mental stress, accumulation of fatigue, and immunosuppression in a healthy human having a sense of fatigue, in particular, a sense of fatigue caused by a mental load or a sense of fatigue caused by a mental load and a physical load.

### [3-3] Temporal change and amount of change of oxidation markers

Figure 9 shows the temporal change and amount of change of oxidation markers at pre-ingestion (before load application) and after 8-week ingestion. As can be seen from Figure 9, no significant difference was found for any of the creatinine level, BAP level, d-ROMs level, and d-ROMs/BAP ratio.

These results suggested that the astaxanthin and/or astaxanthin-containing extract effects, in a mode without any statistically significant antioxidative effect, exerts, to improve dull-headedness, improve decreased motivation, improve depressed mood, resolve frustration, reduce feeling of body heaviness, improve decreased vigor and/or activity, enhance feeling of friendliness, improve or enhance the quality of sleep with respect to, for example, sleepiness on rising, initiation and maintenance of sleep, frequent dreaming, and sleep length, reduce, improve, or resolve physical stress, prevent accumulation of mental stress, prevent accumulation of fatigue, and prevent immunosuppression, in a healthy human having a sense of fatigue, in particular, a sense of fatigue caused by a mental load or a sense of fatigue caused by a mental load and a physical load. Hence, it was revealed that the above-described effects to improve dull-headedness, improve decreased motivation, improve depressed mood, resolve frustration, reduce feeling of body heaviness, improve decreased vigor and/or activity, enhance feeling of friendliness, improve or enhance the quality of sleep with respect to, for example, sleepiness on rising, initiation and maintenance of sleep, frequent dreaming, and sleep length, reduce, improve, or resolve physical stress, prevent accumulation of mental stress, prevent accumulation of fatigue, and prevent immunosuppression, in a healthy human having a sense of fatigue, in particular, a sense of fatigue caused by a mental load or a sense of fatigue caused by a mental load and a physical load by ingestion of the astaxanthin and/or astaxanthin-containing extract are not necessarily associated with oxidation and antioxidation in the human body.

### [3-4] Blood (serum) astaxanthin level

Data for the subjects and blood (serum) astaxanthin (AX) levels at pre-ingestion (before load application), after 4-week ingestion, after 8-week ingestion, and after 12-week ingestion in the subjects are shown in Figure 10; changes in VAS (plots for the placebo group) at each measurement point from before load application for the factors of "Clear-headedness", "Concentration", "Motivation", "Mood", and "Frustration" after 8-week ingestion are shown in (C), (F), (I), and (L) in Figures 11 to 15; correlations between change in VAS in the astaxanthin group at each measurement point from before load application for the factors of "Clear-headedness", "Concentration", "Motivation", "Mood", and "Frustration" and blood (serum) astaxanthin level after 8-week ingestion are shown in (A), (D), (G), and (J) in Figures 11 to 15; changes (plots for the placebo group) from pre-ingestion (before load application) in the factors of "Sleepiness on rising", "Initiation and maintenance of sleep", "Frequent dreaming", "Refreshing", and "Sleep length" after 12-week ingestion are shown in (C), (F), (I), (L), and (O) in Figure 16; and correlations between change in each factor from pre-ingestion (before load application) in the astaxanthin group and blood (serum) astaxanthin level after 12-week ingestion are shown in (A), (D), (G), (J), and (M) in Figure 16.

As can be seen from (C), (F), (I), and (L) in Figures 11 to 15, (A), (D), (G), and (J) in Figures 11 to 15, (C), (F), (I), (L), and (O) in Figure 16, and (A), (D), (G), (J), and (M) in Figure 16, difference was found for effects to improve dull-headedness, improve decreased concentration, improve decreased motivation, improve depressed mood, resolve frustration, and improve or enhance the quality of sleep with respect to, for example, sleepiness on rising, initiation and maintenance of sleep, frequent dreaming, and sleep length, for a healthy human having a sense of fatigue, in particular, a sense of fatigue caused by a mental load or a sense of fatigue caused by a mental load and a physical load. More specifically, it was revealed that effects to improve dull-headedness, improve decreased concentration, improve decreased motivation, improve depressed mood, resolve frustration, and improve or enhance the quality of sleep with respect to, for example, sleepiness on rising, initiation and maintenance of sleep, frequent dreaming, and sleep length, for a healthy human having, in particular, a sense of fatigue caused by a mental load or a sense of fatigue caused by a mental load and a physical load are exerted when astaxanthin is ingested by (administered to) a subject in need thereof in a dosage achieving a human blood astaxanthin level equal to approximately 0 ng/mL to approximately 160 ng/mL in terms of the free form of astaxanthin or an equivalent dosage thereof. Any of the factors in Figures 11 to 15 was more effective after the rest period for a mental load than after mental load application, more effective after the rest period for a mental load than after application of both loads, more effective after the rest period for both loads than after application of both loads, and more effective after the rest period for a mental load or after the rest period for both loads than after mental load application.

That is, these results revealed that one example of the mode without any statistically significant antioxidative effect exerted by the astaxanthin and/or astaxanthin-containing extract is the blood (serum) astaxanthin level.

### [3-5] Quantity of astaxanthin ingested per kg of human body weight per day

As described in [5-5] in <Study 1>, body weight measurement was performed at pre-ingestion (before load application), after 4-week ingestion, after 8-week ingestion, and after 12-week ingestion. Data for the subjects and body weights of the subjects at pre-ingestion (before load application), after 4-week ingestion, after 8-week ingestion, and after 12-week ingestion are shown in Figure 10; changes in VAS (plots for the placebo group) at each measurement point from before load application for the factors of "Clear-headedness", "Concentration", "Motivation", "Mood", and "Frustration" after 8-week ingestion are shown in (C), (F), (I), and (L) in Figures 11 to 15; correlations between change in each factor from before load application in the astaxanthin group and the quantity of astaxanthin ingested per kg of human body weight per day after 8-week ingestion are shown in (B), (E), (H), and (K) in Figures 11 to 15; changes (plots for the placebo group) in the factors of "Sleepiness on rising", "Initiation and maintenance of sleep", "Frequent dreaming", and "Refreshing" from pre-ingestion (before load application) after 12-week ingestion are shown in (C), (F), (I), (L), and (O) in Figure 16; and correlations between change in each factor from pre-ingestion (before load application) in the astaxanthin group and the quantity of astaxanthin ingested per kg of human body weight per day after 12-week ingestion are shown in (B), (E), (H), (K), and (N) in Figure 16.

As can be seen from (C), (F), (I), and (L) in Figures 11 to 15, (B), (E), (H), and (K) in Figures 11 to 15, (C), (F), (I), (L), and (O) in Figure 16, and (B), (E), (H), (K), and (N) in Figure 16, it was revealed that effects to improve dull-headedness, improve decreased concentration, improve decreased motivation, improve depressed mood, resolve frustration, and improve or enhance the quality of sleep with respect to, for example, sleepiness on rising, initiation and maintenance of sleep, frequent dreaming, and sleep length, for a healthy human having a sense of fatigue, in particular, a sense of fatigue caused by a mental load or a sense of fatigue caused by a mental load and a physical load are exerted when astaxanthin is ingested by (administered to) a subject in need thereof in a dosage equal to approximately 0.1 mg to approximately 0.3 mg kg of body weight per day in terms of the free form of astaxanthin or an equivalent dosage thereof.

That is, these results revealed that one example of the mode without any statistically significant antioxidative effect exerted by the astaxanthin and/or astaxanthin-containing extract is the quantity of astaxanthin ingested per kg of human body weight per day.

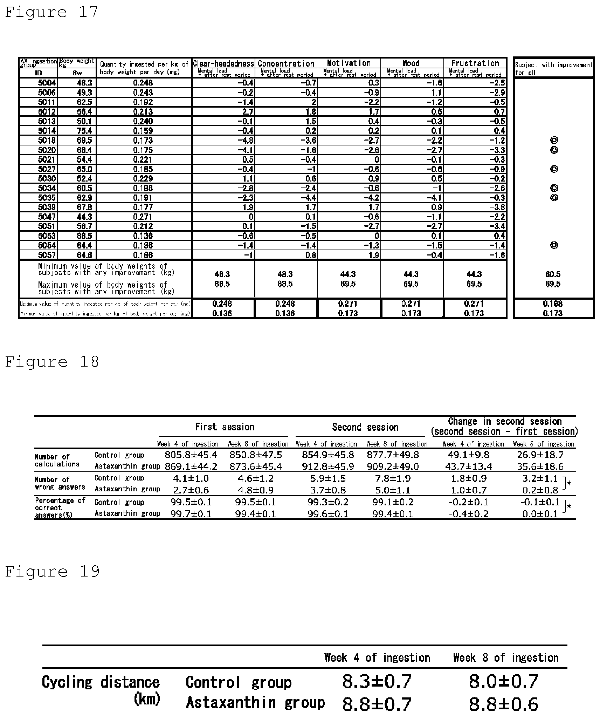

In addition, the relation between the quantity of astaxanthin ingested per kg of human body weight per day measured after 8-week ingestion and load-induced change calculated on the basis of VAS (Visual Analog Scale) scores before load application and after the rest period for a mental load (calculation load; Uchida-Kraepelin test) after 8-week ingestion was examined. One capsule of the test food (120 mg of Haematococcus algae extract and 10 mg of tocotrienol mixture derived from palm oil) was ingested for each body weight measurement. Figure 17 shows the results. As shown in Figure 17, the minimum value and maximum value of the body weights of subjects who exhibited improvement or enhancement in the factor of "Clear-headedness" were 48.3 kg and 88.5 kg, respectively; the minimum value and maximum value of the body weights of subjects who exhibited improvement or enhancement in the factor of "Concentration" were 48.3 kg and 88.5 kg, respectively; the minimum value and maximum value of the body weights of subjects who exhibited enhancement in the factor of "Motivation" were 44.3 kg and 69.5 kg, respectively; the minimum value and maximum value of the body weights of subjects who exhibited improvement or enhancement in the factor of "Mood" were 44.3 kg and 69.5 kg, respectively; and the minimum value and maximum value of the body weights of subjects who exhibited improvement, reduction, or resolution in the factor of "Frustration" were 44.3 kg and 69.5 kg, respectively.

That is, the quantity of astaxanthin ingested per kg of human body weight per day (as the free form of astaxanthin) which provided improvement or resolution in the factor of "Sense of mental fatigue" was 0.159 mg to 0.248 mg; the quantity of astaxanthin ingested per kg of human body weight per day which provided improvement or enhancement in the factor of "Clear-headedness" was 0.136 mg to 0.248 mg; the quantity of astaxanthin ingested per kg of human body weight per day which provided improvement or enhancement in the factor in "Concentration" was 0.136 mg to 0.248 mg; the quantity of astaxanthin ingested per kg of human body weight per day which provided enhancement of the factor in "Motivation" was 0.173 mg to 0.271 mg; the quantity of astaxanthin ingested per kg of human body weight per day which provided improvement or enhancement in the factor of "Mood" was 0.173 mg to 0.271 mg; and the quantity of astaxanthin ingested per kg of human body weight per day which provided improvement, reduction, or resolution in the factor of "Frustration" was 0.173 mg to 0.271 mg.

These results revealed that one example of the mode without any statistically significant antioxidative effect exerted by the astaxanthin and/or astaxanthin-containing extract is the quantity of astaxanthin ingested per kg of human body weight per day (as the free form of astaxanthin). Specifically, it was revealed that effects to improve dull-headedness, improve decreased concentration, improve decreased motivation, improve depressed mood, and resolve frustration for a healthy human having a sense of fatigue, in particular, a sense of fatigue caused by a mental load or a sense of fatigue caused by a mental load and a physical load are exerted when astaxanthin is ingested by (administered to) a subject in need thereof in a dosage equal to approximately 0.1 mg to approximately 0.3 mg per kg of body weight per day in terms of the free form of astaxanthin or an equivalent dosage thereof. More specifically, it was revealed that effects to improve dull-headedness, improve decreased concentration, improve decreased motivation, improve depressed mood, and resolve frustration for a healthy human having a sense of fatigue, in particular, a sense of fatigue caused by a mental load or a sense of fatigue caused by a mental load and a physical load are exerted when astaxanthin is ingested by (administered to) a subject in need thereof in a dosage equal to approximately 0.1 mg to approximately 0.3 mg per kg of body weight per day in terms of the free form of astaxanthin or an equivalent dosage thereof.

That is, these results revealed that one example of the mode without any statistically significant antioxidative effect exerted by the astaxanthin and/or astaxanthin-containing extract is the ingestion of astaxanthin per kg of human body weight per day.

In terms of ingestion of astaxanthin per kg of human body weight per day and ingestion of tocotrienol per kg of human body weight per day, effects to improve dull-headedness, improve decreased concentration, improve decreased motivation, improve depressed mood, and resolve frustration for a healthy human having a sense of fatigue, in particular, a sense of fatigue caused by a mental load or a sense of fatigue caused by a mental load and a physical load are exerted when astaxanthin and tocotrienol are ingested respectively in a dosage equal to approximately 0.06 mg to approximately 0.14 mg per kg of body weight per day in terms of the free form of astaxanthin or an equivalent dosage thereof and in a dosage equal to approximately 0.11 mg to approximately 0.23 mg per kg of body weight per day or an equivalent dosage thereof. More specifically, effects to improve dull-headedness, improve decreased concentration, improve decreased motivation, improve mood disorders for a human having a sense of fatigue, improve depressed mood, and resolve frustration for a healthy human having a sense of fatigue, in particular, a sense of fatigue caused by a mental load or a sense of fatigue caused by a mental load and a physical load are exerted when astaxanthin and tocotrienol are ingested by (administered to) a subject in need thereof respectively in a dosage equal to approximately 0.06 mg to approximately 0.14 mg per kg of body weight per day in terms of the free form of astaxanthin or an equivalent dosage thereof and in a dosage equal to approximately 0.11 mg to approximately 0.23 mg per kg of body weight per day or an equivalent dosage thereof.

### 4. Evaluation of task performance

Figure 18 shows the results of the continuous calculation task to apply a mental load. As shown in Figure 18, after 8-week ingestion, the astaxanthin group exhibited significant improvement in percentage of correct answers and change in the number of wrong answers in the second session in comparison with the control group. Figure 19 shows the results of the cycling task to apply a physical load. As shown in Figure 19, no significant difference was observed in cycling distance between the groups either after 4-week ingestion or after 8-week ingestion.

As expected from these results, the improvement of a sense of mental fatigue in combination with the enhancement of task performance strongly supports the anti-mental fatigue effect of astaxanthin.

Astaxanthin is known to inhibit oxidative modification of carnitine palmitoyl transferase-1 (CPT-1), which is a rate-limiting enzyme for lipolysis and involved in incorporation of long- and medium-chain fatty acids, on the mitochondrial outer membrane at a cell level. In addition, this inhibition is known to cause acceleration of mitochondrial β-oxidation to promote use of lipids as an energy substrate during exercise in an animal which ingested astaxanthin, resulting in saving of saccharides as compensation, suppression of reduction of glycogen in the muscle and liver, and prevention of decreased blood glucose. Muscle glycogen is an energy source during exercise, and astaxanthin is believed to have an effect to reduce muscle fatigue through inhibiting depletion of energy substrates, and reduction of glycogen in the brain is known to be involved in mental fatigue. Accordingly, the present inventors infer that the effect of astaxanthin to save glycogen in the living body acts to suppress both physical fatigue and mental fatigue.

### <Results 2>

### Results of temporal change of human blood (plasma) astaxanthin level through long-term ingestion of astaxanthin in Study 2

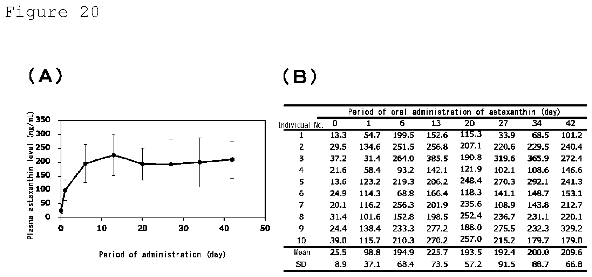

Figure 20 shows the results. As can be seen from Figure 20, when a food containing 60 mg of Haematococcus algae extract (6 mg as the free form of astaxanthin) and 270 mg of edible fat and oil per capsule was ingested at 2 capsules/day for 42 days, the human blood (plasma) astaxanthin level plateaued at approximately 200 ng/mL about six days after the initiation of ingestion. From the results, it was revealed that the human blood (plasma) astaxanthin level was not lowered at least as long as ingestion of an astaxanthin-containing food was continued.

### <Adverse events>

Although one subject who had failed to come to the clinic on the day of assessment after entry to the study was withdrawn, none of the subjects complained of adverse subjective symptoms throughout the study period. Medical examination by a physician also revealed no remarkable abnormalities. No adverse event has been previously found even in the case that 12 mg/day of astaxanthin and 40 mg/day of tocotrienol are ingested for eight weeks, and no adverse event was found also in this study.

## Claims

1. A food-and-drink composition comprising astaxanthin and/or astaxanthin-containing extract as an active ingredient as one or more selected from the following (a), (b), (c), (d), (e), (f), and (g):
(a) a food-and-drink composition for improving dull-headedness in a human having a sense of fatigue;
(b) a food-and-drink composition for improving decreased concentration in a human having a sense of fatigue;
(c) a food-and-drink composition for improving decreased motivation in a human having a sense of fatigue;
(d) a food-and-drink composition for improving depressed mood in a human having a sense of fatigue;
(e) a food-and-drink composition for resolving frustration in a human having a sense of fatigue;
(f) a food-and-drink composition for reducing feeling of body heaviness in a human having a sense of fatigue; and
(g) a food-and-drink composition for improving decreased vigor and/or activity in a human having a sense of fatigue.

2. A food-and-drink composition comprising astaxanthin and/or astaxanthin-containing extract as an active ingredient as one or more selected from the following (a), (b), (c), (d), and (e):
(a) a food-and-drink composition for improving dull-headedness in a human having a sense of fatigue to be ingested by (administered to) a subject in need thereof in a dosage achieving a human blood astaxanthin level equal to approximately 0 to approximately 160 ng/mL in terms of a free form of astaxanthin or an equivalent dosage thereof;
(b) a food-and-drink composition for improving decreased concentration in a human having a sense of fatigue to be ingested by (administered to) a subject in need thereof in a dosage achieving a human blood astaxanthin level equal to approximately 0 to approximately 160 ng/mL in terms of a free form of astaxanthin or an equivalent dosage thereof;
(c) a food-and-drink composition for improving decreased motivation in a human having a sense of fatigue to be ingested by (administered to) a subject in need thereof in a dosage achieving a human blood astaxanthin level equal to approximately 0 to approximately 160 ng/mL in terms of a free form of astaxanthin or an equivalent dosage thereof;
(d) a food-and-drink composition for improving depressed mood in a human having a sense of fatigue to be ingested by (administered to) a subject in need thereof in a dosage achieving a human blood astaxanthin level equal to approximately 0 to approximately 160 ng/mL in terms of a free form of astaxanthin or an equivalent dosage thereof; and
(e) a food-and-drink composition for resolving frustration in a human having a sense of fatigue to be ingested by (administered to) a subject in need thereof in a dosage achieving a human blood astaxanthin level equal to approximately 0 to approximately 160 ng/mL in terms of a free form of astaxanthin or an equivalent dosage thereof.

3. A food-and-drink composition comprising astaxanthin and/or astaxanthin-containing extract as an active ingredient as one or more selected from the following (a), (b), (c), (d), and (e):
(a) a food-and-drink composition for improving dull-headedness in a human having a sense of fatigue to be ingested by (administered to) a subject in need thereof in a dosage equal to approximately 0.1 to approximately 0.3 mg per kg of body weight per day in terms of a free form of astaxanthin or an equivalent dosage thereof;
(b) a food-and-drink composition for improving decreased concentration in a human having a sense of fatigue to be ingested by (administered to) a subject in need thereof in a dosage equal to approximately 0.1 to approximately 0.3 mg per kg of body weight per day in terms of a free form of astaxanthin or an equivalent dosage thereof;
(c) a food-and-drink composition for improving decreased motivation in a human having a sense of fatigue to be ingested by (administered to) a subject in need thereof in a dosage equal to approximately 0.1 to approximately 0.3 mg per kg of body weight per day in terms of a free form of astaxanthin or an equivalent dosage thereof;
(d) a food-and-drink composition for improving depressed mood in a human having a sense of fatigue to be ingested by (administered to) a subject in need thereof in a dosage equal to approximately 0.1 to approximately 0.3 mg per kg of body weight per day in terms of a free form of astaxanthin or an equivalent dosage thereof; and
(e) a food-and-drink composition for resolving frustration in a human having a sense of fatigue to be ingested by (administered to) a subject in need thereof in a dosage equal to approximately 0.1 to approximately 0.3 mg per kg of body weight per day in terms of a free form of astaxanthin or an equivalent dosage thereof.

4. The food-and-drink composition according to any one of claims 1 to 3, wherein the sense of fatigue is a sense of fatigue caused by a mental load or a sense of fatigue caused by a mental load and a physical load.

5. The food-and-drink composition according to any one of claims 1 to 4, further comprising at least any of tocotrienol and olive oil.

6. Astaxanthin and/or astaxanthin-containing extract for use for at least any application of the following (a), (b), (c), (d), (e), (f), and (g):
(a) improvement of dull-headedness in a human having a sense of fatigue;
(b) improvement of decreased concentration in a human having a sense of fatigue;
(c) improvement of decreased motivation in a human having a sense of fatigue;
(d) improvement of depressed mood in a human having a sense of fatigue;
(e) resolution of frustration in a human having a sense of fatigue;
(f) reduction of feeling of body heaviness in a human having a sense of fatigue; and
(g) improvement of decreased vigor and/or activity in a human having a sense of fatigue.

7. The astaxanthin and/or an astaxanthin-containing extract according to claim 6, for use as a food-and-drink.

8. A method for improving, reduce or resolving at least any of the following (a), (b), (c), (d), (e), (f), and (g) through administration of astaxanthin:
(a) dull-headedness in a human having a sense of fatigue;
(b) decreased concentration in a human having a sense of fatigue;
(c) decreased motivation in a human having a sense of fatigue;
(d) depressed mood in a human having a sense of fatigue;
(e) frustration in a human having a sense of fatigue;
(f) feeling of body heaviness in a human having a sense of fatigue; and
(g) decreased vigor and/or activity in a human having a sense of fatigue.
